# EUROPEAN PATENT APPLICATION

(11) **EP 3 067 061 A1**
(43) Date of publication of application: **14.09.2016**
(21) Application number: 16150396.6
(22) Date of filing: 11.08.2010
(51) Int. Cl.: A61K 38/04, A61K 38/07

(54) **METHODS FOR PREVENTING OR TREATING METABOLIC SYNDROME**

(30) Priority: 12.08.2009 US 233275 P
(62) Divisional of application: 10808688.5
(71) Applicant: Cornell University, Ithaca, NY 14850 (US)
(72) Inventor: SZETO, Hazel, H, New York, NY New York 10128 (US)
(74) Representative: Atkinson, Jennifer

(57) **Abstract**

The Invention provides methods of preventing or treating metabolic syndrome in a mammalian subject. The methods comprise administering to the subject an effective amount of an aromatic cationic peptide to subjects in need thereof.

## Description

### CROSS-REFERENCE TO RELATED APPLICATIONS

This application claims priority to U.S. Provisional Patent Application No. 61/233,275, filed August 12, 2009, the entire contents of which are hereby incorporated by reference in its entirety.

### TECHNICAL FIELD

The present technology relates generally to the methods of preventing or treating metabolic syndrome. In particular, the present technology relates to administering aromatic-cationic peptides in effective amounts to prevent or treat metabolic syndrome or associated conditions in mammalian subjects.

### BACKGROUND

The following description is provided to assist the understanding of the reader. None of the information provided or references cited is admitted to be prior art to the present invention.

Metabolic syndrome is a collection of health disorders or risks that increase the chance of developing heart disease, stroke, and diabetes. The condition is also known by other names, including syndrome X and dysmetabolic syndrome. Metabolic syndrome may include any of a variety of underlying metabolic phenotypes, including insulin resistance and/or obesity predisposition phenotypes.

Metabolic syndrome is often characterized by any of a number of metabolic disorders or risk factors, which are generally considered to most typify metabolic syndrome when more than one of these factors are present in a single individual. The factors include: central obesity (disproportionate fat tissue in and around the abdomen), atherogenic dyslipidemia (these include a family of blood fat disorders including, *e.g*., high triglycerides, low HDL cholesterol, and high LDL cholesterol that can foster plaque buildups in the vascular system, including artery walls), high blood pressure, insulin resistance or glucose intolerance (the inability to properly use insulin or blood sugar), a chronic prothrombotic state (*e.g*., characterized by high fibrinogen or plasminogen activator inhibitor levels in the blood), and a chronic proinflammatory state (*e.g*., characterized by higher than normal levels of high-sensitivity C-reactive protein in the blood). People with metabolic syndrome are at increased risk of coronary heart disease, other diseases related to plaque buildups in artery walls (*e.g*., stroke and peripheral vascular disease) and type 2 diabetes.

The underlying causes of metabolic syndrome are unclear--though certain effects of the disorder such as obesity and lack of physical activity are often causal in nature. Given inheritance patterns for the disorder, there also appear to be genetic factors that underlie the syndrome.

### SUMMARY

The present technology relates generally to the treatment or prevention of metabolic syndrome and associated conditions in mammals through administration of therapeutically effective amounts of aromatic-cationic peptides to subjects in need thereof. In particular embodiments, the aromatic-cationic peptides treat or prevent metabolic syndrome by reducing the severity or occurrence of dyslipidemia, central obesity, blood fat disorders, or insulin resistance.

In one aspect, the disclosure provides a method of treating or preventing metabolic syndrome and associated conditions in a mammalian subject in need thereof, comprising administering to the mammalian subject a therapeutically effective amount of an aromatic-cationic peptide. In some embodiments, the aromatic-cationic peptide is a peptide having:
at least one net positive charge;
a minimum of four amino acids;
a maximum of about twenty amino acids;
a relationship between the minimum number of net positive charges (pₘ) and the total number of amino acid residues (r) wherein 3pₘ is the largest number that is less than or equal to r + 1; and a relationship between the minimum number of aromatic groups (a) and the total number of net positive charges (pₜ) wherein 2a is the largest number that is less than or equal to pₜ + 1, except that when a is 1, pₜ may also be 1. In particular embodiments, the mammalian subject is a human.

In one embodiment, 2pₘ is the largest number that is less than or equal to r+1, and a may be equal to pₜ. The aromatic-cationic peptide may be a water-soluble peptide having a minimum of two or a minimum of three positive charges.

In one embodiment, the peptide comprises one or more non-naturally occurring amino acids, for example, one or more D-amino acids. In some embodiments, the C-terminal carboxyl group of the amino acid at the C-terminus is amidated. In certain embodiments, the peptide has a minimum of four amino acids. The peptide may have a maximum of about 6, a maximum of about 9, or a maximum of about 12 amino acids.

In one embodiment, the peptide comprises a tyrosine or a 2',6'-dimethyltyrosine (Dmt) residue at the N-terminus. For example, the peptide may have the formula Tyr-D-Arg-Phe-Lys-NH₂ (SS-01) or 2',6'-Dmt-D-Arg-Phe-Lys-NH₂ (SS-02). In another embodiment, the peptide comprises a phenylalanine or a 2',6'-dimethylphenylalanine residue at the N-terminus. For example, the peptide may have the formula Phe-D-Arg-Phe-Lys-NH₂ (SS-20) or 2',6'-Dmp-D-Arg-Phe-Lys-NH₂. In a particular embodiment, the aromatic-cationic peptide has the formula D-Arg-2',6'-Dmt-Lys-Phe-NH₂ (referred to herein as SS-31, MTP-131, or Bendavia™)_{.}

In one embodiment, the peptide is defined by formula I: 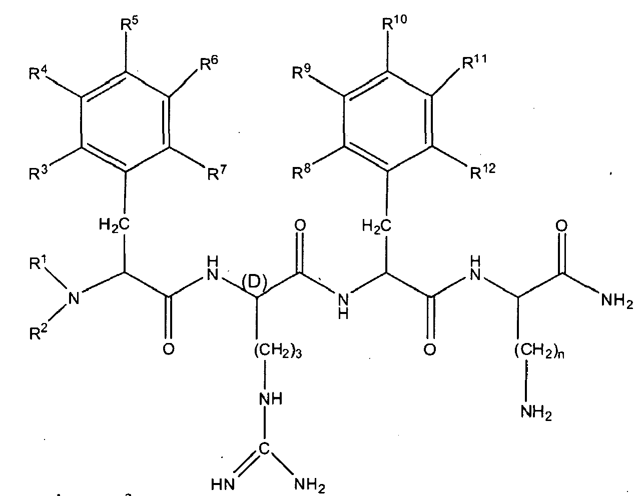 wherein R¹ and R² are each independently selected from
(i) hydrogen;
(ii) linear or branched C₁-C₆ alkyl;
(iii) 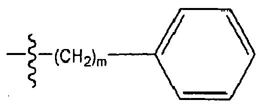 where m = 1-3;
(iv) 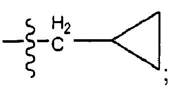
(v) 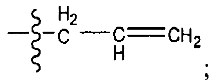
R³, R⁴, R⁵, R⁶, R⁷, R⁸, R⁹, R¹⁰, R¹¹ and R¹² are each independently selected from
(i) hydrogen;
(ii) linear or branched C₁-C₆ alkyl;
(iii) C₁-C₆ alkoxy;
(iv) amino;
(v) C₁-C₄ alkylamino;
(vi) C₁-C₄ dialkylamino;
(vii) nitro;
(viii) hydroxyl;
(ix) halogen, where "halogen" encompasses chloro, fluoro, bromo, and iodo; and n is an integer from 1 to 5.

In a particular embodiment, R¹, R², R³, R⁴, R⁵, R⁶, R⁷, R⁸, R⁹, R¹⁰, R¹¹, and R¹² are all hydrogen; and n is 4. In another embodiment, R¹, R², R³, R⁴, R⁵, R⁶, R⁷, R⁸, R⁹, and R¹¹ are all hydrogen; R⁸ and R¹² are methyl; R¹⁰ is hydroxyl; and n is 4.

In one embodiment, the peptide is defined by formula II: 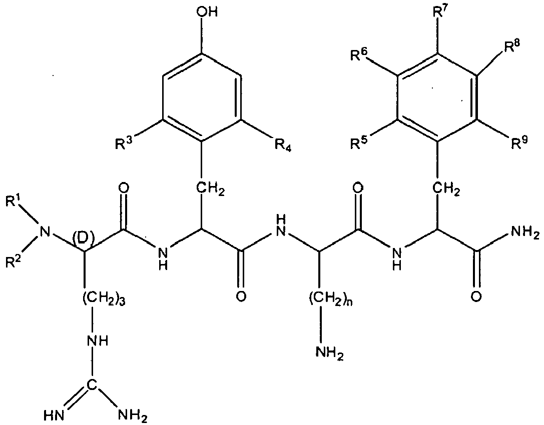
wherein R¹ and R² are each independently selected from
   (i) hydrogen;
   (ii) linear or branched C₁-C₆ alkyl;
   (iii) 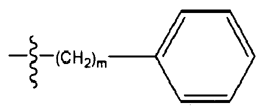
   (iv) 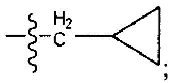
   (v) 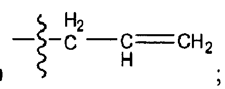 where m = 1-3;
R³ and R⁴ are each independently selected from
   (i) hydrogen;
   (ii) linear or branched C₁-C₆ alkyl;
   (iii) C₁-C₆ alkoxy;
   (iv) amino;
   (v) C₁-C₄ alkylamino;
   (vi) C₁-C₄ dialkylamino;
   (vii) nitro;
   (viii) hydroxyl;
   (ix) halogen, where "halogen" encompasses chloro, fluoro, bromo, and iodo;
R⁵, R⁶, R⁷, R⁸, and R⁹ are each independently selected from
   (i) hydrogen;
   (ii) linear or branched C₁-C₆ alkyl;
   (iii) C₁-C₆ alkoxy;
   (iv) amino;
   (v) C₁-C₄ alkylamino;
   (vi) C₁-C₄ dialkylamino;
   (vii) nitro;
   (viii) hydroxyl;
   (ix) halogen, where "halogen" encompasses chloro, fluoro, bromo, and iodo; and n is an integer from 1 to 5.

In a particular embodiment, R¹ and R² are hydrogen; R³ and R⁴ are methyl; R⁵, R⁶, R⁷, R⁸, and R⁹ are all hydrogen; and n is 4.

In some embodiments, the metabolic syndrome is associated with resistance to insulin-mediated glucose uptake, glucose intolerance, hyperinsulemia, increased LDL-cholesterol, increased VLDL, increased triglycerides, decreased HDL-cholesterol, increased plasminogen activator inhibitor-1 (PAI-1) levels and hypertension. In an illustrative embodiment, the metabolic syndrome is characterized by three or more of the following criteria:
(a) abdominal obesity: waist circumference > 102 cm in men and >88 cm in women;
(b) hypertriglyceridemia: ≥ 150 mg/dl (1.695 mmol/l);
(c) low HDL cholesterol: <40 mg/dl (1.036 mmol/l) in men and <50 mg/dl (1.295 mmol/l) in women;
(d) high blood pressure: ≥ 130/85 mm Hg; and
(e) high fasting glucose: ≥ 110 mg/dl (>6.1 mmol/l).

In an illustrative embodiment, the metabolic syndrome is characterized by diabetes, impaired glucose tolerance, impaired fasting glucose, or insulin resistance plus two or more of the following abnormalities:
(a) high blood pressure: ≥ 160/90 mm Hg;
(b) hyperlipidemia: triglyceride concentration ≥ 150 mg/dl (1.695 mmol/l) and/or HDL cholesterol <35 mg/dl (0.9 mmol/l) in men and <39 mg/dl (1.0 mmol/l) in women;
(c) central obesity: waist-to-hip ratio of >0.90 in men or >0.85 in women or BMI >30 kg/m²; and
(d) microalbuminuria: urinary albumin excretion rate ≥ 20 µg/min or an albumin to creatinine ratio ≥ 20 mg/g.

In an illustrative embodiment, the metabolic syndrome is characterized by three or more of the following criteria:
(a) triglycerides >150 mg/dl;
(b) systolic blood pressure (BP) ≥ 130 mm Hg or diastolic blood pressure ≥ 85 mm Hg or on antihypertensive treatment;
(c) high-density lipoprotein cholesterol <40 mg/dl;
(d) fasting blood sugar (FBS) >110 mg/dl; and
(e) body mass index > 28.8 kg/m².

In one embodiment, the metabolic syndrome is treated through improvement of lipid metabolism compared to the subject's lipid metabolism before being administered the peptide. In one embodiment, the improvement in lipid metabolism is reduction of blood triglyceride level compared to the subject's blood triglyceride level before being administered the peptide. In one embodiment, the improvement in lipid metabolism is improvement of blood HDL/LDL cholesterol ratio compared to the subject's blood HDL/LDL cholesterol before being administered the peptide. In one embodiment, the metabolic syndrome is treated by reducing blood sugar level compared to the subject's blood sugar level before being administered the peptide. In one embodiment, the metabolic syndrome is treated by a reduction in body weight compared to the subject's body weight before being administered the peptide.

In some embodiments, the aromatic-cationic peptides are used to treat or prevent conditions associated with metabolic syndrome in mammalian subjects, which include, but are not limited to, central obesity, dyslipidemia, hyperinsulinemia, type II diabetes, abnormal vascular endothelial function, retinopathy, coronary artery disease, cardiovascular disease, renal dysfunction, hypertension, fatty liver, neuropathy, and hyperuricemia. Specific examples of cardiovascular disease potentially caused by metabolic syndrome and associated conditions include myocardial infarction, hemorrhagic or ischemic stroke (cerebral infarction).

The aromatic-cationic peptides may be administered in a variety of ways. In some embodiments, the peptides may be administered orally, topically, intranasally, intravenously, subcutaneously, or transdermally (*e.g*., by iontophoresis).

### BRIEF DESCRIPTION OF THE FIGURES

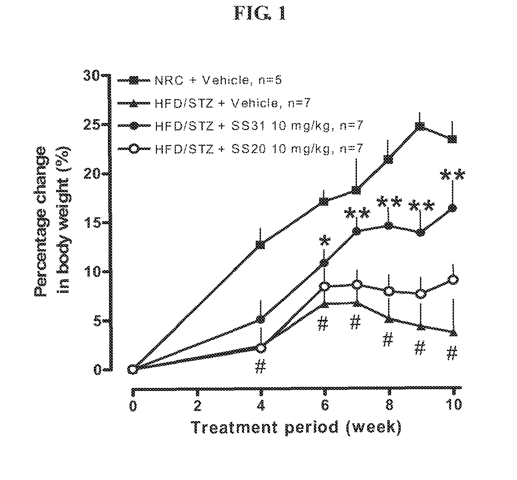

FIG. 1 is a chart illustrating the effects of peptide treatment on body weight. Values are Mean ± S.E.M. # P < 0.001 vs. normal rat chow (NRC) + Vehicle. * P < 0.05, ** P < 0.01 vs. HFD/STZ + Vehicle. Squares, NRC rats treated with vehicle, n= 5; triangles, HFD/STZ rats treated with vehicle, n=7; solid circles, HFD/STZ rats treated with SS-31 at 10 mg/kg, n=7; open circles, HFD/STZ rats treated with SS-20 at 10 mg/kg, n=7.

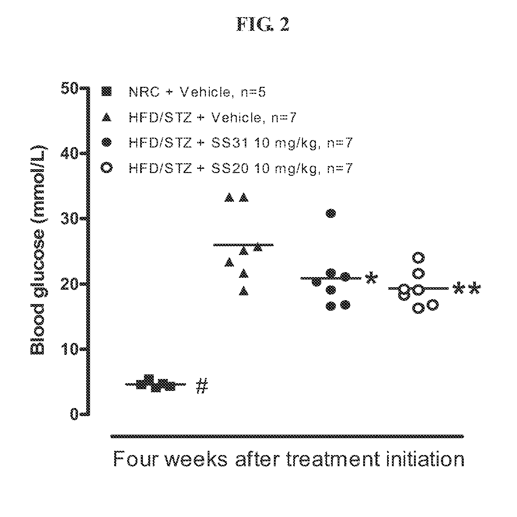

FIG. 2 is a chart illustrating the effects of peptide treatment on blood glucose at 4 weeks of treatment. Values are Mean ± S.E.M. # *P* < 0.001 vs. HFD/STZ + Vehicle. ** P =* 0.031, ** *P* = 0.006 vs. HFD/STZ + Vehicle. Squares, NRC rats treated with vehicle, n= 5; triangles, HFD/STZ rats treated with vehicle, n=7; solid circles, HFD/STZ rats treated with SS-31 at 10 mg/kg, n=7; open circles, HFD/STZ rats treated with SS-20 at 10 mg/kg, n=7.

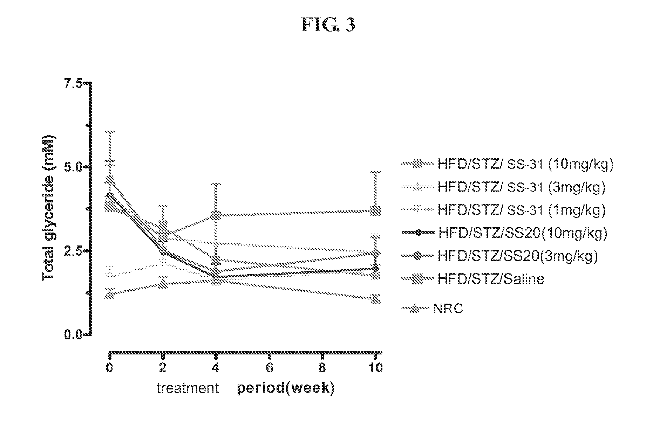

FIG. 3 is a chart illustrating the effects of peptide treatment on total glycerides in HFD/STZ rats over 10 weeks of treatment.

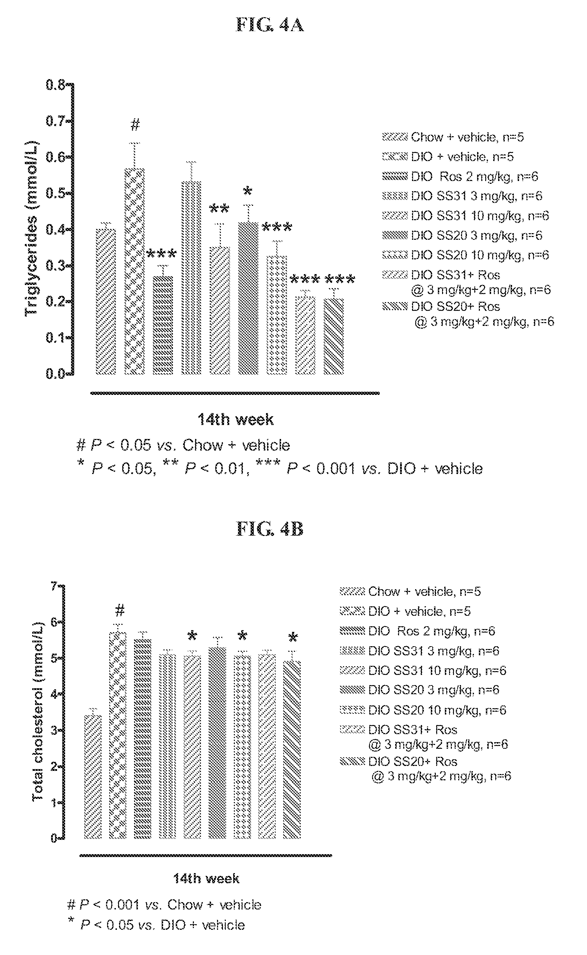

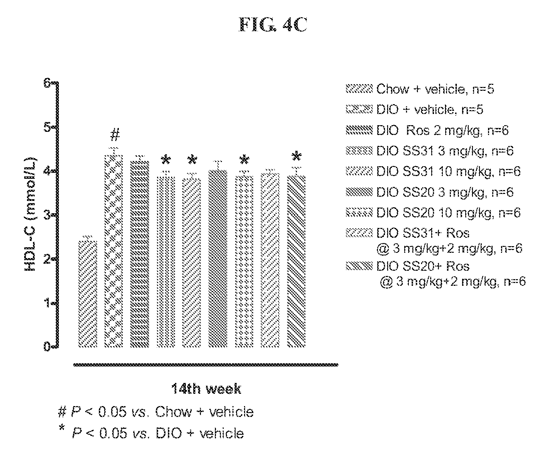

FIG. 4A is a chart illustrating the effects of peptide treatment on triglycerides in a murine model of diet-induced obesity after 14 weeks of treatment. FIG. 4B is a chart illustrating the effects of peptide treatment on total cholesterol in a murine model of diet-induced obesity after 14 weeks of treatment. FIG. 4C is a chart illustrating the effects of peptide treatment on HDL cholesterol in a murine model of diet-induced obesity after 14 weeks of treatment.

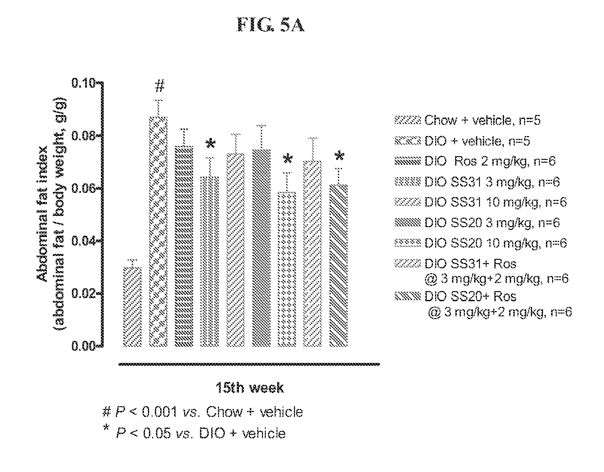

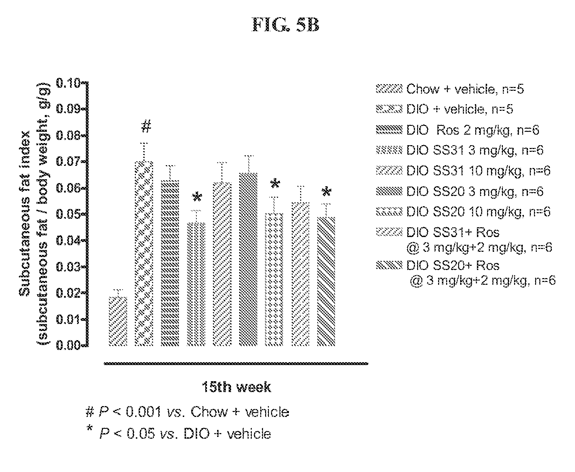

FIG. 5A is a chart illustrating the effects of peptide treatment on abdominal fat in a murine model of diet-induced obesity after 14 weeks of treatment. FIG. 5B is a chart illustrating the effects of peptide treatment on subcutaneous fat in a murine model of diet-induced obesity after 14 weeks of treatment.

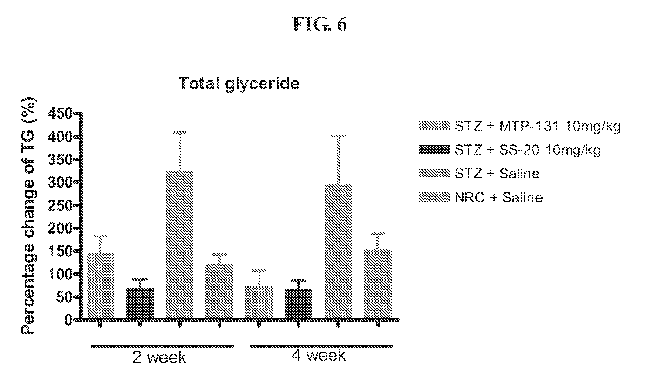

FIG. 6 is a chart illustrating the effects of peptide treatment on glycerides in an STZ rat model of metabolic syndrome.

### DETAILED DESCRIPTION

It is to be appreciated that certain aspects, modes, embodiments, variations and features of the invention are described below in various levels of detail in order to provide a substantial understanding of the present invention.

In practicing the present invention, many conventional techniques in molecular biology, protein biochemistry, cell biology, immunology, microbiology and recombinant DNA are used. These techniques are well-known and are explained in, *e.g.,* Current Protocols in Molecular Biology, Vols. I-III, Ausubel, Ed. (1997); Sambrook et al., Molecular Cloning: A Laboratory Manual, Second Ed. (Cold Spring Harbor Laboratory Press, Cold Spring Harbor, NY, 1989); DNA Cloning: A Practical Approach, Vols. I and II, Glover, Ed. (1985); Oligonucleotide Synthesis, Gait, Ed. (1984); Nucleic Acid Hybridization, Hames & Higgins, Eds. (1985); Transcription and Translation, Hames & Higgins, Eds. (1984); Animal Cell Culture, Freshney, Ed. (1986); Immobilized Cells and Enzymes (IRL Press, 1986); Perbal, A Practical Guide to Molecular Cloning; the series, Meth. Enzymol., (Academic Press, Inc., 1984); Gene Transfer Vectors for Mammalian Cells, Miller & Calos, Eds. (Cold Spring Harbor Laboratory, NY, 1987); and Meth. Enzymol., Vols. 154 and 155, Wu & Grossman, and Wu, Eds., respectively.

The definitions of certain terms as used in this specification are provided below. Unless defined otherwise, all technical and scientific terms used herein generally have the same meaning as commonly understood by one of ordinary skill in the art to which this invention belongs.

As used in this specification and the appended claims, the singular forms "a", "an" and "the" include plural referents unless the content clearly dictates otherwise. For example, reference to "a cell" includes a combination of two or more cells, and the like.

As used herein, "about" will be understood by persons of ordinary skill in the art and will vary to some extent depending upon the context in which it is used. If there are uses of the term which are not clear to persons of ordinary skill in the art, given the context in which it is used, "about" will mean up to plus or minus 10% of the enumerated value.

As used herein, the "administration" of an agent, drug, or peptide to a subject includes any route of introducing or delivering to a subject a compound to perform its intended function. Administration can be carried out by any suitable route, including orally, intranasally, parenterally (intravenously, intramuscularly, intraperitoneally, or subcutaneously), or topically. Administration includes self-administration and the administration by another.

As used herein, the term "amino acid" includes naturally-occurring amino acids and synthetic amino acids, as well as amino acid analogs and amino acid mimetics that function in a manner similar to the naturally-occurring amino acids. Naturally-occurring amino acids are those encoded by the genetic code, as well as those amino acids that are later modified, *e.g*., hydroxyproline, γ-carboxyglutamate, and O-phosphoserine. Amino acid analogs refers to compounds that have the same basic chemical structure as a naturally-occurring amino acid, *i.e.*, an α-carbon that is bound to a hydrogen, a carboxyl group, an amino group, and an R group, *e.g*., homoserine, norleucine, methionine sulfoxide, methionine methyl sulfonium. Such analogs have modified R groups (*e.g*., norleucine) or modified peptide backbones, but retain the same basic chemical structure as a naturally-occurring amino acid. Amino acid mimetics refers to chemical compounds that have a structure that is different from the general chemical structure of an amino acid, but that functions in a manner similar to a naturally-occurring amino acid. Amino acids can be referred to herein by either their commonly known three letter symbols or by the one-letter symbols recommended by the IUPAC-IUB Biochemical Nomenclature Commission.

As used herein, the term "effective amount" refers to a quantity sufficient to achieve a desired therapeutic and/or prophylactic effect, *e.g*., an amount which results in the prevention of, or a decrease in, the symptoms associated with metabolic syndrome. The amount of a composition administered to the subject will depend on the type and severity of the disease and on the characteristics of the individual, such as general health, age, sex, body weight and tolerance to drugs. It will also depend on the degree, severity and type of disease. The skilled artisan will be able to determine appropriate dosages depending on these and other factors. The compositions can also be administered in combination with one or more additional therapeutic compounds. In the methods described herein, the aromatic-cationic peptides may be administered to a subject having one or more signs of metabolic syndrome. Administration of an effective amount of the aromatic-cationic peptides may improve at least one sign or symptom of metabolic syndrome in the subject, *e.g*., body weight, fasting glucose/insulin/free fatty acid, glucose tolerance (OGTT), muscle insulin sensitivity, markers of insulin signaling (*e.g*., Akt-P, IRS-P), serum triglyceride levels, HDL and LDL cholesterol levels, blood pressure, serum fibrinogen or plasminogen activator inhibitor levels, levels of C-reactive protein, mitochondrial function (*e.g*., respiration or H₂O₂ emission), markers of intracellular oxidative stress (*e.g.*, lipid peroxidation, GSH/GSSG ratio or aconitase activity) and mitochondrial enzyme activity. For example, a "therapeutically effective amount" of the aromatic-cationic peptides is meant levels in which the physiological effects of metabolic syndrome are, at a minimum, ameliorated.

An "isolated" or "purified" polypeptide or peptide is substantially free of cellular material or other contaminating polypeptides from the cell or tissue source from which the agent is derived, or substantially free from chemical precursors or other chemicals when chemically synthesized. For example, an isolated aromatic-cationic peptide would be free of materials that would interfere with diagnostic or therapeutic uses of the agent. Such interfering materials may include enzymes, hormones and other proteinaceous and nonproteinaceous solutes.

As used herein, the term "metabolic syndrome" refers to a combination of metabolic disorders or risk factors, which when present in a single individual, may predispose the individual to developing heart disease, stroke, or diabetes.

As used herein, the terms "polypeptide", "peptide" and "protein" are used interchangeably herein to mean a polymer comprising two or more amino acids joined to each other by peptide bonds or modified peptide bonds, *i.e.*, peptide isosteres. Polypeptide refers to both short chains, commonly referred to as peptides, glycopeptides or oligomers, and to longer chains, generally referred to as proteins. Polypeptides may contain amino acids other than the 20 gene-encoded amino acids. Polypeptides include amino acid sequences modified either by natural processes, such as post-translational processing, or by chemical modification techniques that are well known in the art.

The term "subject" as used herein refers to a member of any vertebrate species. The methods of the presently disclosed subject matter are particularly useful for warm-blooded vertebrates. Provided herein is the treatment of mammals such as humans, as well as those mammals of importance due to being endangered, of economic importance (animals raised on farms for consumption by humans) and/or social importance (animals kept as pets or in zoos) to humans. In particular embodiments, the subject is a human.

As used herein, the terms "treating" or "treatment" or "alleviation" refers to both therapeutic treatment and prophylactic or preventative measures, wherein the object is to prevent or slow down (lessen) the targeted pathologic condition or disorder. A subject is successfully "treated" for metabolic syndrome if, after receiving a therapeutic amount of the aromatic-cationic peptides according to the methods described herein, the subject shows observable and/or measurable reduction in or absence of one or more signs and symptoms of metabolic syndrome. For example, treatment may include a reduction in the fasting blood glucose or insulin levels, reduction in body weight, reduction in serum triglyceride levels or HDL and LDL cholesterol levels. It is also to be appreciated that the various modes of treatment or prevention of medical conditions as described are intended to mean "substantial", which includes total but also less than total treatment or prevention, and wherein some biologically or medically relevant result is achieved.

As used herein, "prevention" or "preventing" of a disorder or condition refers to a compound that, in a statistical sample, reduces the occurrence of the disorder or condition in the treated sample relative to an untreated control sample, or delays the onset or reduces the severity of one or more symptoms of the disorder or condition relative to the untreated control sample.

### Metabolic Syndrome

Metabolic syndrome (also called syndrome X) is a syndrome which can be associated with several criteria such as resistance to insulin-stimulated glucose uptake, glucose intolerance, hyperinsulinemia, increase LDL-cholesterol, increased VLDL triglycerides, decreased HDL cholesterol, increased plasminogen activator inhibitor-1 (PAI-1) levels and hypertension. Other metabolic abnormalities that have been considered as part of the syndrome include abnormal weight or weight distribution, inflammation, microalbuminuria, hyperuricemia, and abnormalities of fibrinolysis and of coagulation.

Glucose intolerance is characterized by a pathological state in which the fasting plasma glucose level is less than 140 mg per deciliter and the 30-, 60-, or 90-minute plasma glucose concentration following a glucose tolerance test exceeds 200 mg per deciliter.

Hyperinsulinemia is a condition in which the level of insulin in the blood is higher than normal. Hyperinsulinemia is caused by overproduction of insulin by the body and is related to insulin resistance.

Insulin resistance occurs when the body does not respond to the insulin made by the pancreas and glucose is less able to enter the cells. Subjects with insulin resistance may or may not go on to develop type 2 diabetes. Any of a variety of tests in current use can be used to determine insulin resistance, including: the Oral Glucose Tolerance Test (OGTT), Fasting Blood Glucose (FBG), Normal Glucose Tolerance (NGT), Impaired Glucose Tolerance (IGT), Impaired Fasting Glucose (IFG), Homeostasis Model Assessment (HOMA), the Quantitative Insulin Sensitivity Check Index (QUICKI) and the Intravenous Insulin Tolerance Test (IVITT). *See also*, De Vegt "The 1997 American Diabetes Association criteria versus the 1985 World Health Organization criteria for the diagnosis of abnormal glucose tolerance: poor agreement in the Hoorn Study." Diab Care 1998, 21:1686-1690; Matthews "Homeostasis model assessment: insulin resistance and B-cell function from fasting plasma glucose and insulin concentrations in man." Diabetologia 1985, 28:412-419; Katz "Quantitative Insulin Sensitivity Check Index: A Simple, Accurate Method for Assessing Insulin Sensitivity In Humans." JCE & M, 2000, 85:2402-2410.

Hypertriglyceridemia is defined by elevated triglyceride concentration in the blood. Hyperlipidemia is charcterized by the presence of excess lipids in the blood. All hyperlipidemias (types I, IIb, III, IV and V) except type IIa are characterized by elevated triglyceride levels. Type I hyperlipidemia is characterized by severe elevations in chylomicrons and elevated triglycerides. Because chylomicrons also contain a small amount of cholesterol, serum cholesterol levels also are quite high. Type IIb hyperlipidemia is the classic mixed hyperlipidemia (high cholesterol and triglycerides) caused by elevations in both low-density lipoprotein (LDL) and very low-density lipoprotein (VLDL). Type III hyperlipidemia is also known as dysbetalipoproteinemia, remnant removal disease, or broad-beta disease. Typically, these patients have elevated total cholesterol and triglyceride levels and are easily confused with patients with type IIb hyperlipidemia. Patients with type III hyperlipidemia have elevations in intermediate-density lipoprotein (IDL), a VLDL remnant. Type IV hyperlipidemia is characterized by abnormal elevations of VLDL and triglycerides. Serum cholesterol levels are normal. Type V hyperlipidemia is the combination of types I and IV (elevations of both chylomicrons and VLDL). Serum cholesterol levels typically are elevated, but the LDL cholesterol levels are normal. Given the rarity of type I disease, when elevated triglycerides are noted, the most likely cause is type V hyperlipidemia.

Very low density lipoprotein (VLDL) are large lipoproteins rich in triglycerides which circulate through the blood giving up their triglycerides to fat and muscle tissue until the VLDL remnants are modified and converted into LDL. High density lipoprotein (HDL) are lipoproteins that transport cholesterol in the blood; composed of a high proportion of protein and relatively little cholesterol; high levels are thought to be associated with decreased risk of coronary heart disease and atherosclerosis.

People with the metabolic syndrome are at increased risk for cardiovascular disease and for increased mortality from both cardiovascular disease and other causes. Studies also have found that clustering of risk factors proposed to be part of the metabolic syndrome may increase the risk for coronary heart disease. In addition, components of the metabolic syndrome are risk factors for diabetes. Metabolic syndrome is often characterized by three or more of the following criteria:
1. Abdominal obesity: waist circumference >102 cm in men and >88 cm in women.
2. Hypertriglyceridemia: ≥ 150 mg/dl (1.695 mmol/l).
3. Low HDL cholesterol: <40 mg/dl (1.036 mmol/l) in men and <50 mg/dl (1.295 mmol/l) in women.
4. High blood pressure: . ≥ 130/85 mm Hg.
5. High fasting glucose: ≥110 mg/dl (≥ 6.1 mmol/l).
6. BMI >28.8 k/m².

Metabolic syndrome can also be characterized by diabetes, impaired glucose tolerance, impaired fasting glucose, or insulin resistance plus two or more of the following abnormalities:
1. High blood pressure: ≥ 160/90 mm Hg.
2. Hyperlipidemia: triglyceride concentrations 150 mg/dl (1.695 mmol/l) and/or HDL cholesterol <35 mg/dl (0.9 mmol/l) in men and <39 mg/dl (1.0 mmol/l) in women.
3. Central obesity: waist-to-hip ratio of >0.90 in men or >0.85 in women and/or body mass index (BMI) >30 kg/m².
4. Microalbuminuria: urinary albumin excretion rate ≥ 90 µg/min or an albumin-to-creatinine ratio ≥ 90 mg/g.

The present inventors have discovered that aromatic-cationic peptides can prevent or treat metabolic syndrome in mammalian subjects. In some cases, the metabolic syndrome may be due to a high fat diet or, more generally, over-nutrition and lack of exercise. The aromatic-cationic peptides may reduce one or more signs or symptoms of metabolic syndrome, including, but not limited to, dyslipidemia, central obesity, blood fat disorders, and insulin resistance.

Without intending to limit the invention to a particular mechanism of action, it is believed that loss of mitochondrial integrity and insulin sensitivity stem from a common metabolic disturbance, *i.e.*, oxidative stress. Over-nutrition, particularly from high fat diets may increase mitochondrial reactive oxygen species (ROS) emission and overall oxidative stress, leading to both acute and chronic mitochondrial dysfunction and the development of metabolic syndrome. The aromatic-cationic peptides mitigate these effects, thereby improving mitochondrial function in various body tissues, thus improving one or more of the risk factors associated with metabolic syndrome.

The present technology relates to the reduction of the symptoms of metabolic syndrome by administration of certain aromatic-cationic peptides. The aromatic-cationic peptides are water-soluble and highly polar. Despite these properties, the peptides can readily penetrate cell membranes. The aromatic-cationic peptides typically include a minimum of three amino acids or a minimum of four amino acids, covalently joined by peptide bonds. The maximum number of amino acids present in the aromatic-cationic peptides is about twenty amino acids covalently joined by peptide bonds. Suitably, the maximum number of amino acids is about twelve, about nine, or about six.

The amino acids of the aromatic-cationic peptides can be any amino acid. As used herein, the term "amino acid" is used to refer to any organic molecule that contains at least one amino group and at least one carboxyl group. Typically, at least one amino group is at the α position relative to a carboxyl group. The amino acids may be naturally occurring. Naturally occurring amino acids include, for example, the twenty most common levorotatory (L) amino acids normally found in mammalian proteins, *i.e.*, alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan, (Trp), tyrosine (Tyr), and valine (Val). Other naturally occurring amino acids include, for example, amino acids that are synthesized in metabolic processes not associated with protein synthesis. For example, the amino acids ornithine and citrulline are synthesized in mammalian metabolism during the production of urea. Another example of a naturally occurring amino acid include hydroxyproline (Hyp).

The peptides optionally contain one or more non-naturally occurring amino acids. Optimally, the peptide has no amino acids that are naturally occurring. The non-naturally occurring amino acids may be levorotary (L-), dextrorotatory (D-), or mixtures thereof. Non-naturally occurring amino acids are those amino acids that typically are not synthesized in normal metabolic processes in living organisms, and do not naturally occur in proteins. In addition, the non-naturally occurring amino acids suitably are also not recognized by common proteases. The non-naturally occurring amino acid can be present at any position in the peptide. For example, the non-naturally occurring amino acid can be at the N-terminus, the C-terminus, or at any position between the N-terminus and the C-terminus.

The non-natural amino acids may, for example, comprise alkyl, aryl, or alkylaryl groups not found in natural amino acids. Some examples of non-natural alkyl amino acids include α-aminobutyric acid, β-aminobutyric acid, γ-aminobutyric acid, δ-aminovaleric acid, and ε-aminocaproic acid. Some examples of non-natural aryl amino acids include ortho-, meta, and para-aminobenzoic acid. Some examples of non-natural alkylaryl amino acids include ortho-, meta-, and para-aminophenylacetic acid, and γ-phenyl-β-aminobutyric acid. Non-naturally occurring amino acids include derivatives of naturally occurring amino acids. The derivatives of naturally occurring amino acids may, for example, include the addition of one or more chemical groups to the naturally occurring amino acid.

For example, one or more chemical groups can be added to one or more of the 2', 3', 4', 5', or 6' position of the aromatic ring of a phenylalanine or tyrosine residue, or the 4', 5', 6', or 7' position of the benzo ring of a tryptophan residue. The group can be any chemical group that can be added to an aromatic ring. Some examples of such groups include branched or unbranched C₁-C₄ alkyl, such as methyl, ethyl, n-propyl, isopropyl, butyl, isobutyl, or t-butyl, C₁-C₄ alkyloxy (*i.e.*, alkoxy), amino, C₁-C₄ alkylamino and C₁-C₄ dialkylamino (*e.g.*, methylamino, dimethylamino), nitro, hydroxyl, halo (*i.e.*, fluoro, chloro, bromo, or iodo). Some specific examples of non-naturally occurring derivatives of naturally occurring amino acids include norvaline (Nva) and norleucine (Nle).

Another example of a modification of an amino acid in a peptide is the derivatization of a carboxyl group of an aspartic acid or a glutamic acid residue of the peptide. One example of derivatization is amidation with ammonia or with a primary or secondary amine, *e.g*. methylamine, ethylamine, dimethylamine or diethylamine. Another example of derivatization includes esterification with, for example, methyl or ethyl alcohol. Another such modification includes derivatization of an amino group of a lysine, arginine, or histidine residue. For example, such amino groups can be acylated. Some suitable acyl groups include, for example, a benzoyl group or an alkanoyl group comprising any of the C₁-C₄ alkyl groups mentioned above, such as an acetyl or propionyl group.

The non-naturally occurring amino acids are preferably resistant, and more preferably insensitive, to common proteases. Examples of non-naturally occurring amino acids that are resistant or insensitive to proteases include the dextrorotatory (D-) form of any of the above-mentioned naturally occurring L-amino acids, as well as L- and/or D- non-naturally occurring amino acids. The D-amino acids do not normally occur in proteins, although they are found in certain peptide antibiotics that are synthesized by means other than the normal ribosomal protein synthetic machinery of the cell. As used herein, the D-amino acids are considered to be non-naturally occurring amino acids.

In order to minimize protease sensitivity, the peptides should have less than five, less than four, less than three, or less than two contiguous L-amino acids recognized by common proteases, irrespective of whether the amino acids are naturally or non-naturally occurring. In one embodiment, the peptide has only D-amino acids, and no L-amino acids. If the peptide contains protease sensitive sequences of amino acids, at least one of the amino acids may be a non-naturally-occurring D-amino acid, thereby conferring protease resistance. An example of a protease sensitive sequence includes two or more contiguous basic amino acids that are readily cleaved by common proteases, such as endopeptidases and trypsin. Examples of basic amino acids include arginine, lysine and histidine.

The aromatic-cationic peptides should have a minimum number of net positive charges at physiological pH in comparison to the total number of amino acid residues in the peptide. The minimum number of net positive charges at physiological pH will be referred to below as (pₘ). The total number of amino acid residues in the peptide will be referred to below as (r). The minimum number of net positive charges discussed below are all at physiological pH. The term "physiological pH" as used herein refers to the normal pH in the cells of the tissues and organs of the mammalian body. For instance, the physiological pH of a human is normally approximately 7.4, but normal physiological pH in mammals may be any pH from about 7.0 to about 7.8.

"Net charge" as used herein refers to the balance of the number of positive charges and the number of negative charges carried by the amino acids present in the peptide. In this specification, it is understood that net charges are measured at physiological pH. The naturally occurring amino acids that are positively charged at physiological pH include L-lysine, L-arginine, and L-histidine. The naturally occurring amino acids that are negatively charged at physiological pH include L-aspartic acid and L-glutamic acid.

Typically, a peptide has a positively charged N-terminal amino group and a negatively charged C-terminal carboxyl group. The charges cancel each other out at physiological pH. As an example of calculating net charge, the peptide Tyr-D-Arg-Phe-Lys-Glu-His-Trp-D-Arg has one negatively charged amino acid (*i.e.*, Glu) and four positively charged amino acids (*i.e*., two Arg residues, one Lys, and one His). Therefore, the above peptide has a net positive charge of three.

In one embodiment, the aromatic-cationic peptides have a relationship between the minimum number of net positive charges at physiological pH (pₘ) and the total number of amino acid residues (r) wherein 3pₘ is the largest number that is less than or equal to r + 1. In this embodiment, the relationship between the minimum number of net positive charges (pₘ) and the total number of amino acid residues (r) is as follows:

**TABLE 1. Amino acid number and net positive charges (3pₘ ≤ p+1)**

| | | | | | | | | | | | | | | | | | | |
|---|---|---|---|---|---|---|---|---|---|---|---|---|---|---|---|---|---|---|
| **(r)** | 3 | 4 | 5 | 6 | 7 | 8 | 9 | 10 | 11 | 12 | 13 | 14 | 15 | 16 | 17 | 18 | 19 | 20 |
| **(pₘ)** | 1 | 1 | 2 | 2 | 2 | 3 | 3 | 3 | 4 | 4 | 4 | 5 | 5 | 5 | 6 | 6 | 6 | 7 |

In another embodiment, the aromatic-cationic peptides have a relationship between the minimum number of net positive charges (pₘ) and the total number of amino acid residues (r) wherein 2pₘ is the largest number that is less than or equal to r + 1. In this embodiment, the relationship between the minimum number of net positive charges (pₘ) and the total number of amino acid residues (r) is as follows:

**TABLE 2. Amino acid number and net positive charges (2pₘ ≤ p+1)**

| | | | | | | | | | | | | | | | | | | |
|---|---|---|---|---|---|---|---|---|---|---|---|---|---|---|---|---|---|---|
| **(r)** | 3 | 4 | 5 | 6 | 7 | 8 | 9 | 10 | 11 | 12 | 13 | 14 | 15 | 16 | 17 | 18 | 19 | 20 |
| **(pₘ)** | 2 | 2 | 3 | 3 | 4 | 4 | 5 | 5 | 6 | 6 | 7 | 7 | 8 | 8 | 9 | 9 | 10 | 10 |

In one embodiment, the minimum number of net positive charges (pₘ) and the total number of amino acid residues (r) are equal. In another embodiment, the peptides have three or four amino acid residues and a minimum of one net positive charge, preferably, a minimum of two net positive charges and more preferably a minimum of three net positive charges.

It is also important that the aromatic-cationic peptides have a minimum number of aromatic groups in comparison to the total number of net positive charges (pₜ). The minimum number of aromatic groups will be referred to below as (a). Naturally occurring amino acids that have an aromatic group include the amino acids histidine, tryptophan, tyrosine, and phenylalanine. For example, the hexapeptide Lys-Gln-Tyr-D-Arg-Phe-Trp has a net positive charge of two (contributed by the lysine and arginine residues) and three aromatic groups (contributed by tyrosine, phenylalanine and tryptophan residues).

The aromatic-cationic peptides should also have a relationship between the minimum number of aromatic groups (a) and the total number of net positive charges at physiological pH (pₜ) wherein 3a is the largest number that is less than or equal to pₜ + 1, except that when pₜ is 1, a may also be 1. In this embodiment, the relationship between the minimum number of aromatic groups (a) and the total number of net positive charges (pₜ) is as follows:

**TABLE 3. Aromatic groups and net positive charges (3a ≤ pₜ+1 or a= pₜ=1)**

| | | | | | | | | | | | | | | | | | | | | |
|---|---|---|---|---|---|---|---|---|---|---|---|---|---|---|---|---|---|---|---|---|
| **(pₜ)** | 1 | 2 | 3 | 4 | 5 | 6 | 7 | 8 | 9 | 10 | 11 | 12 | 13 | 14 | 15 | 16 | 17 | 18 | 19 | 20 |
| **(a)** | 1 | 1 | 1 | 1 | 2 | 2 | 2 | 3 | 3 | 3 | 4 | 4 | 4 | 5 | 5 | 5 | 6 | 6 | 6 | 7 |

In another embodiment, the aromatic-cationic peptides have a relationship between the minimum number of aromatic groups (a) and the total number of net positive charges (pₜ) wherein 2a is the largest number that is less than or equal to pₜ + 1. In this embodiment, the relationship between the minimum number of aromatic amino acid residues (a) and the total number of net positive charges (pₜ) is as follows:

**TABLE 4. Aromatic groups and net positive charges (2a ≤ pₜ+1 or a= pH=1)**

| | | | | | | | | | | | | | | | | | | | | |
|---|---|---|---|---|---|---|---|---|---|---|---|---|---|---|---|---|---|---|---|---|
| **(pₜ)** | 1 | 2 | 3 | 4 | 5 | 6 | 7 | 8 | 9 | 10 | 11 | 12 | 13 | 14 | 15 | 16 | 17 | 18 | 19 | 20 |
| **(a)** | 1 | 1 | 2 | 2 | 3 | 3 | 4 | 4 | 5 | 5 | 6 | 6 | 7 | 7 | 8 | 8 | 9 | 9 | 10 | 10 |

In another embodiment, the number of aromatic groups (a) and the total number of net positive charges (pₜ) are equal.

Carboxyl groups, especially the terminal carboxyl group of a C-terminal amino acid, are preferably amidated with, for example, ammonia to form the C-terminal amide. Alternatively, the terminal carboxyl group of the C-terminal amino acid may be amidated with any primary or secondary amine. The primary or secondary amine may, for example, be an alkyl, especially a branched or unbranched C₁-C₄ alkyl, or an aryl amine. Accordingly, the amino acid at the C-terminus of the peptide may be converted to an amido, N-methylamido, N-ethylamido, N,N-dimethylamido, N,N-diethylamido, N-methyl-N-ethylamido, N-phenylamido or N-phenyl-N-ethylamido group. The free carboxylate groups of the asparagine, glutamine, aspartic acid, and glutamic acid residues not occurring at the C-terminus of the aromatic-cationic peptides may also be amidated wherever they occur within the peptide. The amidation at these internal positions may be with ammonia or any of the primary or secondary amines described above.

In one embodiment, the aromatic-cationic peptide is a tripeptide having two net positive charges and at least one aromatic amino acid. In a particular embodiment, the aromatic-cationic peptide is a tripeptide having two net positive charges and two aromatic amino acids.

Aromatic-cationic peptides include, but are not limited to, the following peptide examples:
Lys-D-Arg-Tyr-NH₂
Phe-D-Arg-His
D-Tyr-Trp-Lys-NH₂
Trp-D-Lys-Tyr-Arg-NH₂
Tyr-His-D-Gly-Met
Phe-Arg-D-His-Asp
Tyr-D-Arg-Phe-Lys-Glu-NH₂
Met-Tyr-D-Lys-Phe-Arg
D-His-Glu-Lys-Tyr-D-Phe-Arg
Lys-D-Gln-Tyr-Arg-D-Phe-Trp-NH₂
Phe-D-Arg-Lys-Trp-Tyr-D-Arg-His
Gly-D-Phe-Lys-Tyr-His-D-Arg-Tyr-NH₂
Val-D-Lys-His-Tyr-D-Phe-Ser-Tyr-Arg-NH₂
Trp-Lys-Phe-D-Asp-Arg-Tyr-D-His-Lys
Lys-Trp-D- Tyr-Arg-Asn-Phe-Tyr-D-His-NH₂
Thr-Gly-Tyr-Arg-D-His-Phe-Trp-D-His-Lys
Asp-D-Trp-Lys-Tyr-D-His-Phe-Arg- D-Gly-Lys-NH₂
D-His-Lys-Tyr- D-Phe-Glu- D-Asp- D-His- D-Lys-Arg-Trp-NH₂
Ala-D-Phe-D-Arg-Tyr-Lys-D-Trp-His-D-Tyr-Gly-Phe
Tyr-D-His-Phe- D-Arg-Asp-Lys- D-Arg-His-Trp-D-His-Phe
Phe-Phe-D-Tyr-Arg-Glu-Asp-D-Lys-Arg-D-Arg-His-Phe-NH₂
Phe-Try-Lys-D-Arg-Trp-His-D-Lys-D-Lys-Glu-Arg-D-Tyr-Thr
Tyr-Asp-D-Lys-Tyr-Phe- D-Lys- D-Arg-Phe-Pro-D-Tyr-His-Lys
Glu-Arg-D-Lys-Tyr- D-Val-Phe- D-His-Trp-Arg-D-Gly-Tyr-Arg-D-Met-NH₂
Arg-D-Leu-D-Tyr-Phe-Lys-Glu- D-Lys-Arg-D-Trp-Lys- D-Phe-Tyr-D-Arg-Gly
D-Glu-Asp-Lys-D-Arg-D-His-Phe-Phe-D-Val-Tyr-Arg-Tyr-D-Tyr-Arg-His-Phe-NH₂
Asp-Arg-D-Phe-Cys-Phe-D-Arg-D-Lys-Tyr-Arg-D-Tyr-Trp-D-His-Tyr-D-Phe-Lys-Phe
His-Tyr-D-Arg-Trp-Lys-Phe-D-Asp-Ala-Arg-Cys-D-Tyr-His-Phe-D-Lys-Tyr-His-Ser-NH₂
Gly-Ala-Lys-Phe-D-Lys-Glu-Arg-Tyr-His-D-Arg-D-Arg-Asp-Tyr-Trp-D-His-Trp-His-D-Lys-Asp
Thr-Tyr-Arg-D-Lys-Trp-Tyr-Glu-Asp-D-Lys-D-Arg-His-Phe-D-Tyr-Gly-Val-Ile-D-His-Arg-Tyr-Lys-NH₂

An example of an aromatic-cationic peptide is Phe-D-Arg-Phe-Lys-NH₂ (referred to herein as "SS-20"). An example of another aromatic-cationic peptide is D-Arg-2'6'-Dmt-Lys-Phe-NH₂ (SS-31).

Suitable substitution variants of the peptide include conservative amino acid substitutions. Amino acids may be grouped according to their physicochemical characteristics as follows:
(a) Non-polar amino acids: Ala(A) Ser(S) Thr(T) Pro(P) Gly(G) Cys (C);
(b) Acidic amino acids: Asn(N) Asp(D) Glu(E) Gln(Q);
(c) Basic amino acids: His(H) Arg(R) Lys(K);
(d) Hydrophobic amino acids: Met(M) Leu(L) Ile(I) Val(V); and
(e) Aromatic amino acids: Phe(F) Tyr(Y) Trp(W) His (H).

Substitutions of an amino acid in a peptide by another amino acid in the same group is referred to as a conservative substitution and may preserve the physicochemical characteristics of the original peptide. In contrast, substitutions of an amino acid in a peptide by another amino acid in a different group is generally more likely to alter the characteristics of the original peptide.

Examples of analogs that activate mu-opioid receptors include, but are not limited to, the aromatic-cationic peptides shown in Table 5.

**TABLE 5. Peptide Analogs with Mu-Opioid Activity**

| **Amino Acid Position 1** | **Amino Acid Position 2** | **Amino Acid Position 3** | **Amino Acid Position 4** | **C-Terminal Modification** |
|---|---|---|---|---|
| Tvr | D-Arg | Phe | Lvs | **NH₂** |
| Tvr | D-Arg | Phe | Orn | NH₂ |
| Tyr | D-Arg | Phe | Dab | NH₂ |
| Ty^{r} | D-Arg | Phe | Dap | NH₂ |
| 2'6'Dmt | D-Arg | Phe | Lys | NH₂ |
| 2'6'Dmt | D-Arg | Phe | Lvs-NH(CH₂)₂-NH-dns | NH₂ |
| 2'6'Dmt | D-Arg | Phe | Lys-NH(CH₂)₂-NH-atn | NH₂ |
| 2'6'Dmt | D-Arg | Phe | dnsLys | NH₂ |
| 2'6'Dmt | D-Cit | Phe | Lys | NH₂ |
| 2'6'Dmt | D-Cit | Phe | Ahp | NH₂ |
| 2'6'Dmt | D-Arg | Phe | Orn | NH₂ |
| 2'6'Dmt | D-Arg | Phe | Dab | NH₂ |
| 2'6'Dmt | D-Arg | Phe | Dap | NH₂ |
| 2'6'Dmt | D-Arg | Phe | Ahp(2-aminoheptanoic acid) | NH₂ |
| Bio-2'6'Dmt | D-Arg | Phe | Lys | NH₂ |
| 3'5'Dmt | D-Arg | Phe | Lys | NH₂ |
| 3'5'Dmt | D-Arg | Phe | Orn | NH₂ |
| 3'5'Dmt | D-Arg | Phe | Dab | NH₂ |
| 3'5'Dmt | D-Arg | Phe | Dap | NH₂ |
| Tyr | D-Arg | Tyr | Lys | NH₂ |
| Tyr | D-Arg | Tyr | Orn | NH₂ |
| Tyr | D-Arg | Tyr | Dab | NH₂ |
| Tyr | D-Arg | Tyr | Dap | NH₂ |
| 2'6'Dmt | D-Arg | Tyr | Lvs | NH₂ |
| 2'6'Dmt | D-Arg | Tyr | Orn | NH₂ |
| 2'6'Dmt | D-Arg | Tyr | Dab | NH₂ |
| 2'6'Dmt | D-Arg | Tyr | Dap | NH₂ |
| 2'6'Dmt | D-Arg | 2'6'Dmt | Lys | NH₂ |
| 2'6'Dmt | D-Arg | 2'6'Dmt | Orn | NH₂ |
| 2'6'Dmt | D-Arg | 2'6'Dmt | Dab | NH₂ |
| 2'6'Dmt | D-Arg | 2'6'Dmt | Dap | NH₂ |
| 3'5'Dmt | D-Arg | 3'5'Dmt | Arg | NH₂ |
| 3'5'Dmt | D-Arg | 3'5'Dmt | Lvs | NH₂ |
| 3'5'Dmt | D-Arg | 3'5'Dmt | Orn | NH₂ |
| 3'5'Dmt | D-Arg | 3'5'Dmt | Dab | NH₂ |
| Tyr | D-Lys | Phe | Dap | NH₂ |
| Tvr | D-Lys | Phe | Arg | NH₂ |
| Tyr | D-Lys | Phe | Lys | NH₂ |
| Tyr | D-Lys | Phe | Orn | NH₂ |
| 2'6'Dnri | D-Lys | Phe | Dab | NH₂ |
| 2'6'Dmt | D-Lys | Phe | Dap | NH₂ |
| 2'6'Dmt | D-Lys | Phe | Arg | NH₂ |
| 2'6'Dmt | D-Lys | Phe | Lys | NH₂ |
| 3'5'Dmt | D-Lys | Phe | Orn | NH₂ |
| 3'5'Dmt | D-Lys | Phe | Dab | NH₂ |
| 3'5'Dmt | D-Lys | Phe | Dap | NH₂ |
| 3'5'Dmt | D-Lys | Phe | Arg | NH₂ |
| Tyr | D-Lys | Tyr | Lys | NH₂ |
| Tyr | D-Lys | Tyr | orn | NH₂ |
| Tyr | D-Lys | Tyr | Dab | NH₂ |
| Tyr | D-1 ys | Tyr | Dap | NH₂ |
| 2'6'Dmt | D-Lys | Tvr | Lys | NH₂ |
| 2'6'Dmt | D-Lys | Tyr | Orn | NH₂ |
| 2'6'Dmt | D-Lys | Tyr | Dab | NH₂ |
| 2'6'Dmt | D-Lys | Tyr | Dap | NH₂ |
| 2'6'Dmt | D-Lys | 2'6'Dmt | Lys | NH₂ |
| 2'6'Dmt | D-Lys | 2'6'Dmt | Orn | NH₂ |
| 2'6'Dmt | D-Lys | 2'6'Dmt | Dab | NH₂ |
| 2'6'Dmt | D-Lys | 2'6'Dmt | Dap | NH₂ |
| 2'6'Dmt | D-Arg | Phe | dnsDap | NH₂ |
| 2'6'Dmt | D-Arg | Phe | atnDap | NH₂ |
| 3'5'Dmt | D-Lys | 3'5'Dmt | Lys | NH₂ |
| 3'5'Dmt | D-Lys | 3'5'Dmt | Orn | NH₂ |
| 3'5'Dmt | D-Lys | 3'5'Dmt | Dab | NH₂ |
| 3'5'Dmt | D-Lys | 3'5'Dmt | Dap | NH₂ |
| Tvr | D-Lys | Phe | Arg | NH₂ |
| Tyr | D-Orn | Phe | Arg | NH₂ |
| Tyr | D-Dab | Phe | Arg | NH₂ |
| Tyr | D-Dap | Phe | Arg | NH₂ |
| 2'6'Dmt | D-Arg | Phe | Arg | NH₂ |
| 2'6'Dmt | D-Lys | Phe | Arg | NH₂ |
| 2'6'Dmt | D-Orn | Phe | Arg | NH₂ |
| 2'6'Dmt | D-Dab | Phe | Arg | NH₂ |
| 3'5'Dmt | D-Dap | Phe | Arg | NH₂ |
| 3'5'Dmt | D-Arg | Phe | Arg | NH₂ |
| 3'5'Dmt | D-Lvs | Phe . | Are | NH₂ |
| 3'5'Dmt | D-Orn | Phe | Arg | NH₂ |
| Tyr | D-Lvs | Tyr | Arg | NH₂ |
| Tyr | D-Oni | Tyr | Arg | NH₂ |
| Tyr | D-Dab | Tyr | Arg | NH₂ |
| Tyr | D-Dap | Tyr | Arg | NH₂ |
| 2'6'Dmt | D-Arg | 2'6'Dmt | Arg | NH₂ |
| 2'6'Dmt | D-Lys | 2'6'Dmt | Arg | NH₂ |
| 2'6'Dmt | D-Orn | 2'6'Dmt | Arg | NH₂ |
| 2'6'Dmt | D-Dab | 2'6'Dmt | Arg | NH₂ |
| 3'5'Dmt | D-Dap | 3'5'Dmt | Arg | NH₂ |
| 3'5'Dmt | D-Arg | 3'5'Dmt | Arg | NH₂ |
| 3'5'Dmt | D-Lys | 3'5'Dmt | Arg | NH₂ |
| 3'5'Dmt | D-Orn | 3'5'Dmt | Arg | NH₂ |
| Mmt | D-Arg | Phe | Lvs | NH₂ |
| Mmt | D-Arg | Phe | Orn | NH₂ |
| Mmt | D-Arg | Phe | Dab | NH₂ |
| Mmt | D-Arg | Phe | Dap | NH₂ |
| Tmt | D-Arg | Phe | Lys | NH₂ |
| Tmt | D-warp | Phe | Orn | NH₂ |
| Tmt | D-Arg | Phe | Dab | NH₂ |
| Tmt | D-Arg | Phe | Dap | NH₂ |
| Hmt | D-Arg | Phe | Lys | NH₂ |
| Hmt | D-Arg | Phe | Orn | NH₂ |
| Hmt | D-Arg | Phe | Dab | NH₂ |
| Hmt | D-Arg | Phe | Dap | NH₂ |
| Mmt | D-Lys | Phe | Lys | NH₂ |
| Mmt | D-Lys | Phe | Orn | NH₂ |
| Mmt | D-Lys | Phe | Dab | NH₂ |
| Mmt | D-Tys | Phe | Dap | NH₂ |
| Mmt | D-Lys | Phe | Arg | NH₂ |
| Tmt | D-Lys | Phe | Lys | NH₂ |
| Tmt | D-Lys | Phe | Orn | NH₂ |
| Tmt | D-Lys | Phe | Dab | NH₂ |
| Tmt | D-Lys | Phe | Dap | NH₂ |
| Tmt | D-Lys | Phe | Arg | NH₂ |
| Hmt | D-Lys | Phe | Lys | NH₂ |
| Hmt | D-Lys | Phe | Orn | NH₂ |
| Hmt | D-Lys | Phe | Dab | NH₂ |
| Hmt | D-Lys | Phe | Dap | NH₂ |
| Hmt | D-Lys | Phe | Arg | NH₂ |
| Mmt | D-Lys | Phe | Arg | NH₂ |
| Mmt | D-Orn | Phe | Arg | NH₂ |
| Mmt | D-Dab | Phe | Arg | NH₂ |
| Mmt | D-Dap | Phe | Arg | NH₂ |
| Mmt | D-Arg | Phe | Arg | NH₂ |
| Tmt | D-Lys | Phe | Arg | NH₂ |
| Tmt | D-Orn | Phe | Arg | NH₂ |
| Tmt | D-Dab | Phe | Arg | NH₂ |
| Tmt | D-Dap | Phe | Arg | NH₂ |
| Tmt | D-Arg | Phe | Arg | NH₂ |
| Hmt | D-Lys | Phe | Arg | NH₂ |
| Hmt | D-Orn | Phe | Arg | NH₂ |
| Hmt | D-Dab | Phe | Arg | NH₂ |
| Hmt | D-Dap | Phe | Arg | NH₂ |
| Hmt | D-Arg | Phe | Arg | NH₂ |

| | | | | |
|---|---|---|---|---|
| Dab = diaminobutyric Dap = diaminopropionic acid Dmt = dimethyltyrosine Mmt = 2'-methyltyrosine Tmt = N, 2',6'-trimethyltyrosine Hmt = 2'-hydroxy,6'-methyltyrosine dnsDap = β-dansyl-L-α,β-diaminopropionic acid atnDap = β-anthraniloyl-L-α,β-diaminopropionic acid Bio = biotin | | | | |

Examples of analogs that do not activate mu-opioid receptors include, but are not limited to, the aromatic-cationic peptides shown in Table 6.

**TABLE 6. Peptide Analogs Lacking Mu-Opioid Activity**

| **Amino Acid Position 1** | **Amino Acid Position 2** | **Amino Acid Position 3** | **Amino Acid Position 4** | **Amino Acid Position 5** | **Amino Acid Position 6** | **Amino Acid Position 7** | **C-Terminal Modification** |
|---|---|---|---|---|---|---|---|
| D-Arg | Dmt | Lys | Phe | | | | NH₂ |
| D-Arg | Dmt | Phe | Lys | | | | NH₂ |
| D-Arg | Phe | Lys | Dmt | | | | NH₂ |
| D-Arg | Phe | Dmt | Lys | | | | NH₂ |
| D-Arg | Lys | Dmt | Phe | | | | NH₂ |
| D-Arg | Lys | Phe | Dmt | | | | NH₂ |
| Phe | Lys | Dmt | D-Arg | | | | NH₂ |
| Phe | Lys | D-Arg | Dmt | | | | NH₂ |
| Phe | D-Arg | Phe | Lys | | | | NH₂ |
| Phe | D-Arg | Dmt | Lys | | | | NH₂ |
| Phe | D-Arg | Lys | Dmt | | | | NH₂ |
| Phe | Dmt | D-Arg | Lys | | | | NH₂ |
| Phe | Dmt | Lys | D-Arg | | | | NH₂ |
| Lys | Phe | D-Arg | Dmt | | | | NH₂ |
| Lys | Phe | Dmt | D-Arg | | | | NH₂ |
| Lys | Dmt | D-Arg | Phe | | | | NH₂ |
| Lys | Dmt | Phe | D-Arg | | | | NH₂ |
| Lys | D-Arg | Phe | Dmt | | | | NH₂ |
| Lys | D-Arg | Dmt | Phe | | | | NH₂ |
| D-Arg | Dmt | D-Arg | Phe | | | | NH₂ |
| D-Arg | Dmt | D-Arg | Dmt | | | | NH₂ |
| D-Arg | Dmt | D-Arg | Tyr | | | | NH₂ |
| D-Arg | Dmt | D-Arg | Trp | | | | NH₂ |
| Trp | D-Arg | Phe | Lys | | | | NH₂ |
| Trp | D-Arg | Tyr | Lys | | | | NH₂ |
| Trp | D-Arg | Trp | Lys | | | | NH₂ |
| Trp | D-Arg | Dmt | Lys | | | | NH₂ |
| D-Arg | Trp | Lys | Phe | | | | NH₂ |
| D-Arg | Trp | Phe | Lys | | | | NH₂ |
| D-Arg | Trp | Lys | Dmt | | | | NH₂ |
| D-Arg | Trp | Dmt | Lys | | | | NH₂ |
| D-Arg | Lys | Trp | Phe | | | | NH₂ |
| D-Arg | Lys | Trp | Dmt | | | | NH₂ |
| Cha | D-Arg | Phe | Lys | | | | NH₂ |
| Ala | D-Arg | Phe | Lys | | | | NH₂ |

| | | | | | | | |
|---|---|---|---|---|---|---|---|
| Cha = cyclohexyl alanine | | | | | | | |

The amino acids of the peptides shown in Table 5 and 6 may be in either the L- or the D- configuration.

The peptides may be synthesized by any of the methods well known in the art. Suitable methods for chemically synthesizing the protein include, for example, those described by Stuart and Young in Solid Phase Peptide Synthesis, Second Edition, Pierce Chemical Company (1984), and in Methods Enzymol. 289, Academic Press, Inc, New York (1997).

### Prophylactic and Therapeutic Uses of Aromatic-Cationic Peptides.

*General.* The aromatic-cationic peptides described herein are useful to prevent or treat disease. Specifically, the disclosure provides for both prophylactic and therapeutic methods of treating a subject at risk of (or susceptible to) metabolic syndrome. Metabolic syndrome is generally associated with type II diabetes, coronary artery disease, renal dysfunction, atherosclerosis, obesity, dyslipidemia, and essential hypertension. Accordingly, the present methods provide for the prevention and/or treatment of metabolic syndrome or associated conditions in a subject by administering an effective amount of an aromatic-cationic peptide to a subject in need thereof. For example, a subject can be administered an aromatic-cationic peptide in an effort to improve one or more of the factors contributing to metabolic syndrome.

In one aspect, the technology provides a method of treating or preventing the specific disorders associated with metabolic syndrome, such as obesity, diabetes, hypertension, and hyperlipidemia, in a mammal by administering an aromatic cationic peptide. In certain embodiments, the specific disorder is obesity. In certain embodiments, the specific disorder is dyslipidemia (*i.e.* hyperlipidemia).

*Determination of the Biological Effect of the Aromatic-Cationic Peptide-Based Therapeutic.* In various embodiments, suitable *in vitro* or *in vivo* assays are performed to determine the effect of a specific aromatic-cationic peptide-based therapeutic and whether its administration is indicated for treatment of metabolic syndrome. In various embodiments, *in vitro* assays can be performed with representative cells of the type(s) involved in the subject's disorder, to determine if a given aromatic-cationic peptide-based therapeutic exerts the desired effect upon the cell type(s). Compounds for use in therapy can be tested in suitable animal model systems including, but not limited to rats, mice, chicken, cows, monkeys, rabbits, and the like, prior to testing in human subjects. Similarly, for *in vivo* testing, any of the animal model system known in the art can be used prior to administration to human subjects. Conditions associated with metabolic syndrome can be readily detected by quantifying body weight, fasting glucose/insulin/free fatty acid, glucose tolerance (OGTT), cholesterol and triglyceride levels, blood pressure, *in vitro* muscle insulin sensitivity, markers of insulin signaling (*e.g.*, Akt-P, IRS-P), mitochondrial function (*e.g.*, respiration or H₂O₂ emission), markers of intracellular oxidative stress (*e.g.*, lipid peroxidation, GSH/GSSG ratio or aconitase activity) or mitochondrial enzyme activity. The fasting glucose/insulin/free fatty acid, glucose tolerance (OGTT), cholesterol and triglyceride levels may be measured using standard clinical laboratory techniques as may be found in Tietz Textbook of Clinical Chemistry, fourth edition. Burtis CA and Ashwood ER eds., 2005.

In one embodiment, administration of an aromatic-cationic peptide to a subject exhibiting one or more conditions associated with metabolic syndrome will cause an improvement in one or more of those conditions. For instance, a subject may exhibit at least about 5%, at least about 10%, at least about 20%, or at least about 50% reduction in body weight compared to the subject prior to receiving the aromatic-cationic peptide. In one embodiment, a subject may exhibit at least about 5%, at least about 10%, at least about 20%, or at least about 50% reduction in HDL cholesterol and/or at least about 5%, at least about 10%, at least about 20%, or at least about 50% increase in LDL cholesterol compared to the subject prior to receiving the aromatic-cationic peptide. In one embodiment, a subject may exhibit at least about 5%, at least about 10%, at least about 20%, or at least about 50% reduction in serum triglycerides. In one embodiment, a subject may exhibit at least about 5%, at least about 10%, at least about 20%, or at least about 50% improvement in oral glucose tolerance (OGTT). In some embodiments, the subject may show observable improvement in more than one condition associated with metabolic syndrome.

*Prophylactic Methods.* In one aspect, the invention provides a method for preventing, in a subject, a disease or condition associated with metabolic syndrome in skeletal muscle tissues, by administering to the subject an aromatic-cationic peptide that modulates one or more signs or markers of metabolic syndrome, *e.g*., body weight, serum triglycerides or cholesterol, fasting glucose/insulin/free fatty acid, glucose tolerance (OGTT), *in vitro* muscle insulin sensitivity, markers of insulin signaling (*e.g.*, Akt-P, IRS-P), mitochondrial function (*e.g*., respiration or H₂O₂ emission), markers of intracellular oxidative stress (*e.g*., lipid peroxidation, GSH/GSSG ratio or aconitase activity) or mitochondrial enzyme activity. The fasting glucose/insulin/free fatty acid, glucose tolerance (OGTT), cholesterol and triglyceride levels, *etc.* may be measured using standard clinical laboratory techniques as may be found in Tietz Textbook of Clinical Chemistry, fourth ed., Burtis C A and Ashwood E R eds., 2005.

Subjects at risk for metabolic syndrome can be identified by, *e.g.,* any or a combination of diagnostic or prognostic assays as described herein. In prophylactic applications, pharmaceutical compositions or medicaments of aromatic-cationic peptides are administered to a subject susceptible to, or otherwise at risk of a disease or condition in an amount sufficient to eliminate or reduce the risk, lessen the severity, or delay the outset of the disease, including biochemical, histologic and/or behavioral symptoms of the disease, its complications and intermediate pathological phenotypes presenting during development of the disease. Administration of a prophylactic aromatic-cationic peptide can occur prior to the manifestation of symptoms characteristic of the aberrancy, such that a disease or disorder is prevented or, alternatively, delayed in its progression. Depending upon the type of aberrancy, *e.g*., an aromatic-cationic peptide which acts to enhance or improve mitochondrial function can be used for treating the subject. The appropriate compound can be determined based on screening assays described herein.

*Therapeutic Methods.* Another aspect of the technology includes methods of reducing the symptoms associated with metabolic syndrome in a subject for therapeutic purposes. In therapeutic applications, compositions or medicaments are administered to a subject suspected of, or already suffering from such a disease in an amount sufficient to cure, or at least partially arrest, the symptoms of the disease, including its complications and intermediate pathological phenotypes in development of the disease. As such, the invention provides methods of treating an individual afflicted with metabolic syndrome or a metabolic syndrome-associated disease or disorder.

The present disclosure contemplates combination therapies of the aromatic-cationic peptides with one or more treatments for blood pressure, blood triglyceride levels, or high cholesterol. Treatment for metabolic syndrome, obesity, insulin resistance, high blood pressure, dyslipidemia, *etc.*, can also include a variety of other approaches, including weight loss and exercise, and dietary changes. These dietary changes include: maintaining a diet that limits carbohydrates to 50 percent or less of total calories; eating foods defined as complex carbohydrates, such as whole grain bread (instead of white), brown rice (instead of white), sugars that are unrefined, increasing fiber consumption by eating legumes (for example, beans), whole grains, fruits and vegetables, reducing intake of red meats and poultry, consumption of "healthy" fats, such as those in olive oil, flaxseed oil and nuts, limiting alcohol intake, etc. In addition, treatment of blood pressure, and blood triglyceride levels can be controlled by a variety of available drugs (*e.g*., cholesterol modulating drugs), as can clotting disorders (*e.g.*, via aspirin therapy) and in general, prothrombotic or proinflammatory states. If metabolic syndrome leads to diabetes, there are, of course, many treatments available for this disease.

### Modes of Administration and Effective Dosages

Any method known to those in the art for contacting a cell, organ or tissue with a peptide may be employed. Suitable methods include *in vitro, ex vivo, or in vivo* methods. *In vivo* methods typically include the administration of an aromatic-cationic peptide, such as those described above, to a mammal, preferably a human. When used *in vivo* for therapy, the aromatic-cationic peptides are administered to the subject in effective amounts (*i.e*., amounts that have desired therapeutic effect). The dose and dosage regimen will depend upon the degree of the metabolic syndrome in the subject, the characteristics of the particular aromatic-cationic peptide used, *e.g*., its therapeutic index, the subject, and the subject's history.

The effective amount may be determined during pre-clinical trials and clinical trials by methods familiar to physicians and clinicians. An effective amount of a peptide useful in the methods of the present invention, preferably in a pharmaceutical composition, may be administered to a mammal in need thereof by any of a number of well-known methods for administering pharmaceutical compounds. The peptide may be administered systemically or locally.

The aromatic-cationic peptides described herein can be incorporated into pharmaceutical compositions for administration, singly or in combination, to a subject for the treatment or prevention of a disorder described herein. Such compositions typically include the active agent and a pharmaceutically acceptable carrier. As used herein the term "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

Pharmaceutical compositions are typically formulated to be compatible with its intended route of administration. Examples of routes of administration include parenteral (*e.g*., intravenous, intradermal, intraperitoneal or subcutaneous), oral, inhalation, transdermal (topical), and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic. For convenience of the patient or treating physician, the dosing formulation can be provided in a kit containing all necessary equipment (*e.g*. vials of drug, vials of diluent, syringes and needles) for a treatment course (*e.g*. 7 days of treatment).

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, a composition for parenteral administration must be sterile and should be fluid to the extent that easy syringability exists. It should be stable under the conditions of manufacture and storage and must be preserved against the contaminating action of microorganisms such as bacteria and fungi.

The aromatic-cationic peptide compositions can include a carrier, which can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thiomerasol, and the like. Glutathione and other antioxidants can be included to prevent oxidation. In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, aluminum monostearate or gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, typical methods of preparation include vacuum drying and freeze drying, which can yield a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, *e.g*., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds can be delivered in the form of an aerosol spray from a pressurized container or dispenser which contains a suitable propellant, *e.g.*, a gas such as carbon dioxide, or a nebulizer. Such methods include those described in U.S. Pat. No. 6,468,798.

Systemic administration of a therapeutic compound as described herein can also be by transmucosal or transdermal means. For transmucosal or transdermal administration, penetrants appropriate to the barrier to be permeated are used in the fonnulation. Such penetrants are generally known in the art, and include, for example, for transmucosal administration, detergents, bile salts, and fusidic acid derivatives. Transmucosal administration can be accomplished through the use of nasal sprays. For transdermal administration, the active compounds are formulated into ointments, salves, gels, or creams as generally known in the art. In one embodiment, transdermal administration may be performed my iontophoresis.

A therapeutic protein or peptide can be formulated in a carrier system. The carrier can be a colloidal system. The colloidal system can be a liposome, a phospholipid bilayer vehicle. In one embodiment, the therapeutic peptide is encapsulated in a liposome while maintaining peptide integrity. As one skilled in the art would appreciate, there are a variety of methods to prepare liposomes. *(See* Lichtenberg et al., Methods Biochem. Anal., 33:337-462 (1988); Anselem et al., Liposome Technology, CRC Press (1993)). Liposomal formulations can delay clearance and increase cellular uptake (*See* Reddy, Ann. Pharmacother., 34 (7-8):915-923 (2000)).

The carrier can also be a polymer, *e.g.*, a biodegradable, biocompatible polymer matrix. In one embodiment, the therapeutic peptide can be embedded in the polymer matrix, while maintaining protein integrity. The polymer may be natural, such as polypeptides, proteins or polysaccharides, or synthetic, such as poly α-hydroxy acids. Examples include carriers made of, *e.g*., collagen, fibronectin, elastin, cellulose acetate, cellulose nitrate, polysaccharide, fibrin, gelatin, and combinations thereof. In one embodiment, the polymer is poly-lactic acid (PLA) or copoly lactic/glycolic acid (PGLA). The polymeric matrices can be prepared and isolated in a variety of forms and sizes, including microspheres and nanospheres. Polymer formulations can lead to prolonged duration of therapeutic effect. *(See* Reddy, Ann. Pharmacother., 34 (7-8):915-923 (2000)). A polymer formulation for human growth hormone (hGH) has been used in clinical trials. *(See* Kozarich and Rich, Chemical Biology, 2:548-552 (1998)).

In some embodiments, the therapeutic compounds are prepared with carriers that will protect the therapeutic compounds against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylacetic acid. Such formulations can be prepared using known techniques. The materials can also be obtained commercially, *e.g*., from Alza Corporation and Nova Pharmaceuticals, Inc. Liposomal suspensions (including liposomes targeted to specific cells with monoclonal antibodies to cell-specific antigens) can also be used as pharmaceutically acceptable carriers. These can be prepared according to methods known to those skilled in the art, for example, as described in U.S. Pat. No. 4,522,811.

The therapeutic compounds can also be formulated to enhance intracellular delivery. For example, liposomal delivery systems are known in the art, see, *e.g*., Chonn and Cullis, "Recent Advances in Liposome Drug Delivery Systems," Current Opinion in Biotechnology 6:698-708 (1995); Weiner, "Liposomes for Protein Delivery: Selecting Manufacture and Development Processes," Immunomethods 4 (3) 201-9 (1994); and Gregoriadis, "Engineering Liposomes for Drug Delivery: Progress and Problems," Trends Biotechnol. 13 (12):527-37 (1995). Mizguchi et al., Cancer Lett. 100:63-69 (1996), describes the use of fusogenic liposomes to deliver a protein to cells both *in vivo* and *in vitro.*

Dosage, toxicity and therapeutic efficacy of the therapeutic agents can be determined by standard pharmaceutical procedures in cell cultures or experimental animals, *e.g.*, for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the methods, the therapeutically effective dose can be estimated initially from cell culture assays. A dose can be formulated in animal models to achieve a circulating plasma concentration range that includes the IC50 (*i.e.*, the concentration of the test compound which achieves a half-maximal inhibition of symptoms) as determined in cell culture. Such information can be used to more accurately determine useful doses in humans. Levels in plasma may be measured, for example, by high performance liquid chromatography.

Typically, an effective amount of the aromatic-cationic peptides, sufficient for achieving a therapeutic or prophylactic effect, range from about 0.000001 mg per kilogram body weight per day to about 10,000 mg per kilogram body weight per day. Preferably, the dosage ranges are from about 0.0001 mg per kilogram body weight per day to about 100 mg per kilogram body weight per day. For example dosages can be 1 mg/kg body weight or 10 mg/kg body weight every day, every two days or every three days or within the range of 1-10 mg/kg every week, every two weeks or every three weeks. In one embodiment, a single dosage of peptide ranges from 0.1-10,000 micrograms per kg body weight. In one embodiment, aromatic-cationic peptide concentrations in a carrier range from 0.2 to 2000 micrograms per delivered milliliter. An exemplary treatment regime entails administration once per day or once a week. In therapeutic applications, a relatively high dosage at relatively short intervals is sometimes required until progression of the disease is reduced or terminated, and preferably until the subject shows partial or complete amelioration of symptoms of disease. Thereafter, the patient can be administered a prophylactic regime.

In some embodiments, a therapeutically effective amount of an aromatic-cationic peptide may be defined as a concentration of peptide at the target tissue of 10⁻¹¹ to 10⁻⁶ molar, *e.g*., approximately 10⁻⁷ molar. This concentration may be delivered by systemic doses of 0.001 to 100 mg/kg or equivalent dose by body surface area. The schedule of doses would be optimized to maintain the therapeutic concentration at the target tissue, most preferably by single daily or weekly administration, but also including continuous administration (*e.g*., parenteral infusion or transdermal application).

In some embodiments, the dosage of the aromatic-cationic peptide is provided at a "low," "mid," or "high" dose level. In one embodiment, the low dose is provided from about 0.0001 to about 0.5 mg/kg/h, suitably from about 0.01 to about 0.1 mg/kg/h. In one embodiment, the mid-dose is provided from about 0.1 to about 1.0 mg/kg/h, suitably from about 0.1 to about 0.5 mg/kg/h. In one embodiment, the high dose is provided from about 0.5 to about 10 mg/kg/h, suitably from about 0.5 to about 2 mg/kg/h.

The skilled artisan will appreciate that certain factors may influence the dosage and timing required to effectively treat a subject, including but not limited to, the severity of the disease or disorder, previous treatments, the general health and/or age of the subject, and other diseases present. Moreover, treatment of a subject with a therapeutically effective amount of the therapeutic compositions described herein can include a single treatment or a series of treatments.

The mammal treated in accordance present methods can be any mammal, including, for example, farm animals, such as sheep, pigs, cows, and horses; pet animals, such as dogs and cats; laboratory animals, such as rats, mice and rabbits. In a preferred embodiment, the mammal is a human.

### EXAMPLES

The present invention is further illustrated by the following examples, which should not be construed as limiting in any way.

### Example 1 - Treatment of Metabolic Syndrome in Rats Fed a High Fat Diet

The effects of aromatic-cationic peptides in treating a subject suffering from metabolic syndrome were investigated in a Sprague-Dawley rat model over a four week period. The example describes the results of such experiments.

*In vivo models.* A rat model of metabolic syndrome was established by combination of 6-week HFD and low-dose STZ (40 mg/kg) injection in SD rats. Rats of the same batch fed with normal chow (NRC) were used as a control.

*Study groups.* Group A: NRC + PBS s.c. q.d. (Mon-Fri)^{*}, n=5; Group B:HFD + STZ + PBS s.c. q.d. (Mon-Fri), n=8; Group C:HFD + STZ + SS-31 10 mg/kg s.c. q.d. (Mon-Fri), n=8; Group D: HFD + STZ + SS-20 10 mg/kg s.c. q.d. (Mon-Fri), n=8. The therapeutic schedule is shown in Table 7.

**TABLE 7. Therapeutic schedule and experimental protocol**

| **Duration** | **Objective** | **Diabetic groups (B, C, D)** | **Control group (A)** |
|---|---|---|---|
| 1st week | Acclimation | Normal rat chow | |
| 2nd - 7th week | Diet manipulation | High fat diet | Normal rat chow |
| End of 7th week | STZ injection | STZ 40 mg/kg, i.p., once | Citrate buffer 2 mL/kg, i.p., once |
| 8th - 12th week | Induction of diabetes | Continue with high fat diet | Continue with normal rat chow |
| 13th - 22nd week | Peptide treatment | Group B: PBS 2 mL/kg/day | Group A: |
| | | Group C: SS-31 10 mg/kg/day | PBS 2 mL/kg/day |
| | | Group D: SS-20 10 mg/kg/day | s.c., 5 days per week |
| | | s.c., 5 days per week | |
| 23rd week | Harvested organs and tissues | | |

*Preparation and administration of test compounds.* SS-31 or SS-20 was dissolved in 0.01 M PBS (pH 7.0). The 10-week treatment was given once a day (5 days a week) with s.c. injection site alternated. The concentration of SS-31 and SS-20 was 5.0 mg/mL. The injection volume (0.2 mL/100 g) for each test animal was adjusted according to body weight taken on the beginning of each week. STZ was dissolved in 0.1 M citrate buffer (pH 4.5) to concentration of 20 mg/mL. STZ is injected intraperitoneally.

*Blood glucose.* Blood glucose was tested by ACCU-CHEK® Advantage™ blood glucose meter and test strips from Roche diagnostics GmbH (Mannheim, Germany).

*Blood sample handling.* Blood samples were collected by tail snip. In some cases when tail snip failed to provide sufficient volume, blood samples were collected from retro-orbital sinus. Collected blood samples were set at room temperature for 30 min to allow clotting before being centrifuged at 1,200 x g for 10 min. The supernatant were collected and re-centrifuged at 1,200 x g for another 5 min to generate the serum. Serum samples were aliquotted and stored at -20°C until being used in analysis.

*Statistics:* Statistic analysis of t-test and one-way ANOVA was performed by SPSS (Statistical Package for Social Science) analysis software.

In accordance with the experimental protocol just described, the effects of the aromatic-cationic peptides in treating conditions associated with metabolic syndrome in a SD rat model were demonstrated. The results are as follows:

*Effects of SS-31 or SS-20 on the body weight of diabetic rats.* Body weight was increased by about 23.3% in NRC group and body weight gain stayed as low as 3.6% in HFD/STZ control rats over the 10-week treatment period (FIG. 1). In contrast, rats treated with SS-31 exhibited a gradual recovery in body weight (P < 0.05 at 6th week and P < 0.01 since 7th to 10th week), which increased by 16.3% by the end of 10th week. Rats treated with SS-20 showed a similar trend of improvement in body weight (9.0% by the end of 10th week).

*Effects of SS-31 or SS-20 on the blood glucose of diabetic rats.* As compared to NRC rats, blood glucose in HFD/STZ vehicle rats remained significantly high during the treatment period. Rats treated with SS-31 and SS-20 showed a down-regulation effect on blood glucose at week 4 post peptide treatment (P < 0.05 and P < 0.01 respectively vs. HFD/STZ vehicle rats, FIG. 2).

In summary, these findings collectively establish that aromatic-cationic peptides, either prevent or compensate for the metabolic dysfunction that develops with over nutrition. As such, administration of the aromatic-cationic peptides is useful in methods of preventing or treating conditions associated with metabolic syndrome in mammalian subjects.

### Example 2 - Effects of SS-20 and SS-31 on Metabolic Syndrome

This study was initiated to investigate the effects of SS-31 and SS-20 in a model of metabolic syndrome over a 10 week period. Rats were fed with a high-fat diet for 6 weeks and then administered a single dose of STZ (30 mg/kg). The rats were maintained on a HFD until 14 weeks after STZ administration. Control rats fed normal rat chow (NRC) for 6 weeks were then administered citrate buffer without STZ. After 5 months, diabetic rats were treated with SS-31 (10 mg/kg, 3 mg/kg, 1 mg/kg s.c. once daily), SS-20 (10 mg/kg, 3 mg/kg s.c. once daily) or vehicle (saline) 5 days a week for 10 weeks. The study groups were as follows:
Group A: HFD/STZ+SS-31 10 mg/kg s.c. q.d. (Mon-Fri.), n=12;
Group B: HFD/STZ+ SS-31 3 mg/kg s.c. q.d. (Mon-Fri.), n=12;
Group C: HFD/STZ+ SS-31 1 mg/kg s.c. q.d. (Mon-Fri.), n=10;
Group D: HFD/STZ+SS-20 10 mg/kg s.c. q.d. (Mon-Fri.), n=10;
Group E: HFD/STZ+SS-20 3 mg/kg s.c. q.d. (Mon-Fri.), n=10;
Group F: HFD/STZ+ vehicle s.c. q.d. (Mon-Fri.), n=10;
Group G: NRC + vehicle s.c. q.d. (Mon-Fri.), n=10.

**TABLE 8. Therapeutic Schedule**

| **Duration** | **Objective** | **Groups A,B, C, D, E, F** | **Control group (G)** |
|---|---|---|---|
| 1^{st} week | Acclimation | Normal rat chow | |
| 2^{nd} - 7^{th} week | Diet manipulation | High fat diet | Normal rat chow |
| End of 7^{th} week | STZ injection | STZ 30 mg/kg, i.p., once | Citrate buffer 2 mL/kg, i.p., once |
| 8^{th} - 27^{th} week | Induction of diabetes | Continue with high fat diet-21th week stop | Continue with normal rat chow |
| | | Group A: MTP-131 10mg/kg/day | |
| | | Group B: MTP-131 3mg/kg/day | |
| 28^{th} - 37^{th} week | Peptide treatment | Group C: MTP-131 1mg/kg/day | Group G: |
| | | Group D: SS-20 10mg/kg/day | PBS 2 mL/kg/day |
| | | Group E: SS-20 3mg/kg/day | s.c., 5 days per week |
| | | Group F: PBS 2mL/kg/day | |
| | | s.c., 5 days per week | |
| 38^{th} week | Harvested organs and tissues | | |

HFD feeding for 6 weeks produced obvious body weight gain (452.9 ± 3.8g in HFD group vs. 425.5 ± 7.1 g, P<0.01) and STZ administration increased blood glucose (26.5 ± 0.5 mmol/L in HFD/STZ rats vs. 9.8 ± 0.5 mmol/L in NRC rats, P< 0.001) and hyperlipidemia (P < 0.01), indicating a metabolic syndrome-like disorder in these test subjects. Hence, the experimental model was successfully established for metabolic syndrome.

During the 10-week period of peptide treatment, no obvious changes were found in body weight and blood glucose in either the SS-31 or SS-20 groups. The blood glucose of NRC group stayed in normal range, while that of STZ treatment groups remained higher than 20 mmol/L throughout 10-week period of peptide treatment (FIG. 3).

FIG. 3 shows that TG level of HFD/STZ rats was much higher than NRC rats before peptide treatment. After 10 weeks' peptides treatment, TG level was decreased in the SS-31 and SS-20-treated groups compared to HFD/STZ group. TG level of rats in SS-31 and SS-20 treatment groups was decreased from 3.77 ± 0.38 mM to 1.75 ± 0.33 mM in SS-31 10 mg/kg group, from 4.17 ± 0.88 mM to 2.46 ± 0.52 mM in SS-31 3mg/kg group, from 4.17 ± 1.02 mM to 197 ± 0.46 mM in SS-20 10mg/kg group, from 4.66 ± 1.40 mM to 2.43 ± 0.47 mM in SS-20 3mg/kg group, from 3.90 ± 0.65 mM to 3.70 ± 1.16 mM in SS-20 3mg/kg group. But TG level of rats in HFD/STZ group was not decreased (3.90 ± 0.65 mM vs. 3.70 ± 1.16 mM). Therefore, SS-31 and SS-20 have beneficial effects on lipid metabolism.

### Example 3 - Effects of SS-20 and SS-31 on Diet-Induced Obesity

To analyze the pharmacodynamic effects of SS-31 and SS-20 against diet-induced obesity (DIO) in C57BL/6 mice, the test subjects were fed either a normal diet or a high-fat diet having the composition shown in Table 9.

**TABLE 9. Diet Composition**

| Ingredients | NRC | | High fat diet (HFD) | |
|---|---|---|---|---|
| | *gm*% | *kcal%* | *gm*% | *kcal%* |
| Protein | 19 | 20 | 26 | 20 |
| Carbohydrate | 67 | 70 | 26 | 20 |
| Fat (Lard) | 4 | 10 | 35 | 60 |

After several days of acclimation, animals were weighted and randomly assigned to the following treatment groups.

**TABLE 10. Treatment Groups**

| **Group** | **Animal Type** | **Animal #** | **Treatment** | **Dosage** | **Route** |
|---|---|---|---|---|---|
| A | Regular | 10 | Vehicle | (N/A) | s.c. |
| B | DIO | 10 | Vehicle | (N/A) | s.c. |
| C | DIO | 12 | Rosiglitazone | 2 mg/kg | p.o |
| D | DIO | 12 | SS-31 | 3 mg/kg | s.c. |
| E | DIO | 12 | SS-31 | 10 mg/kg | s.c. |
| F | DIO | 12 | SS-20 | 3 mg/kg | s.c. |
| G | DIO | 12 | SS-20 | 10 mg/kg | s.c. |
| H | DIO | 12 | SS-31 + Rosiglitazone | 3 mg/kg + 2 mg/kg | s.c. + p.o. |
| I | DIO | 12 | SS-20 + Rosiglitazone | 3 mg/kg + 2 mg/kg | s.c. + p.o. |

Starting from the first day of treatment, animals in Groups B to I were switched to HFD. During the entire treatment period, the following activities were performed on specific days.

**TABLE 11. Therapeutic Schedule**

| **Activity** | | **Date Performed** |
|---|---|---|
| Body Weight Measurement | | Every Week Starting from Dav 1 |
| Blood Sample Collection | TC Measurement | Day 0, 14, 29, 87, 103 (fast) |
| | TG Measurement | Day 103 (fast) |
| | HDL Measurement | Day 103 (fast) |
| | LDL Measurement | Day 103 (fast) |
| | ALT Measurement | Dav 103 (fast) |
| | Insulin Measurement | (TBD) |
| Blood Glucose Level Measurement | Fast | Day 8, 22, 93 |
| | Post-Prandial | Day 87, 99 |
| IPGTT | | Day 36, 100 |
| IPITT | | Day 43, 107 |
| Animal Sacrificing * | Fat Weight | Day 23, 109 |
| | Liver Weight | Day 23, 109 |
| | Creatinine Measurement | Day 23 |
| | Kidney weight | Day 109 |
| | Other Tissue Harvest | Day 109 |

| | | |
|---|---|---|
| * On Day 23, four animals were sacrificed from all groups. All the remaining animals were sacrificed on the last day of in-life study (Day 109). | | |

*Dose Preparation* & *Administration.* The treatment was given once a day (5 days a week) with injection site alternated. The concentration of SS-31 and SS-20 dosed at 3 mg/kg was 0.6 mg/ml and that of 10 mg/kg was 2 mg/ml. The treatment volume (0.05 ml/10 g for s.c. and 0.2 ml/10 g for p.o.) for each test animal was adjusted according to its body weight taken on the beginning of each week.

*Blood Sample Collection.* Blood samples were collected by tail snip according to the Medicilon/MPI Preclinical Research (Shanghai) LLC SOP on Blood Collection Techniques. In some cases when tail snip failed to provide sufficient volume, blood samples were also collected from retro-orbital sinus. Collected blood samples were set at room temperature for 1 hour to allow clotting before being centrifuged at 2,000 x g for 10 min. The supernatant were collected and re-centrifuged at 2,000 x g for another 10 min. to generate the serum. Part of the serum samples were used for total cholesterol (TC) analysis immediately. The rest were stored at -20°C until being used in insulin analysis.

*Serum Insulin, Triglyceride (TG) & Total Cholesterol (TC*). Serum insulin levels were measured using Rat/Mouse Insulin ELISA Kit from Millipore Corporation (Catalog # EZRMI-13K, St. Charles, Missouri, USA). Serum TG levels were measured by automatic biochemical analyzer. FIG. 5A is a chart illustrating the effects of peptide treatment on triglycerides in a murine model of diet-induced obesity after 14 weeks of treatment. FIG. 5B is a chart illustrating the effects of peptide treatment on total cholesterol in a murine model of diet-induced obesity after 14 weeks of treatment. FIG. 5C is a chart illustrating the effects of peptide treatment on HDL cholesterol in a murine model of diet-induced obesity after 14 weeks of treatment.

*IPGTT.* IPGTT was performed according to the Medicilon-MPI Preclinical Research (Shanghai) LLC SOP on Intraperitoneal (IP) Glucose Tolerance Test with slight modifications. Mice were fast overnight before the baseline glucose levels assessed in the next morning. After that, an IP injection of 1 g/kg D-glucose was given to each animal and the blood glucose levels were then measured on 15, 30, 60 and 120 minutes post the IP injection. The results are shown in Table 12 below, and indicate that SS-20 and SS-31 significantly improve glucose tolerance in the obese mouse model.

**TABLE 12. Glucose Tolerance**

| Group | n | Week 5 |
|---|---|---|
| Chow + vehicle | 6 | 1213.6 ± 54.1 |
| DIO = vehicle | 6 | 1719.7 ±56.8^{###} |
| DIO Rosi @ 2mg/kg | 8 | 1487.5± 44.2 * |
| DIO SS31 @ 3mg/kg | 8 | 1460.6 ± 64.1 ** |
| DIO SS31 @ 10mg/kg | 8 | 1584.3 ± 78.3 |
| DIO SS20 @ 3mg/kg | 8 | 1534.1 ± 53.3 * |
| DIO SS20 @ 10mg/kg | 8 | 1494.5 ± 54.4 * |
| DIO SS31+ Rosiglitazone @ 3mg/kg+2mg/kg | 8 | 1597.3 ± 54.0 |
| DIO SS20+ Rosiglitazone @ 3mg/kg+2mg/kg | 8 | 1673.8 ± 63.7 |
| ### *P* < 0.001 *vs.* Chow + vehicle | | |
| * *P* < 0.05, ** *P* < 0.01 *vs.* DIO + vehicle | | |

FIG. 5A is a chart illustrating the effects of peptide treatment on abdominal fat after 14 weeks of treatment. FIG. 5B is a chart illustrating the effects of peptide treatment on subcutaneous fat after 14 weeks of treatment. The results show that the treatment with SS-31 or SS-20 is capable of reducing the abdominal fat index and subcutaneous fat index of the test subjects.

### Example 4- Effects of SS-31 and SS-20 on Metabolic Syndrome in STZ Diabetes Model

The objective of this study was to explore the protective effect of SS-31 or SS-20 against Streptozotocin (STZ) induced diabetic complications and metabolic syndrome in male Sprague-Dawley rats.

Rat models of type 1 diabetes were established by administration of high-dose STZ (30 mg/kg). Rats of the same batch fed with regular chow are used as normal control. The study groups included: Group A: STZ+SS-31 10 mg/kg s.c. q.d. (M.-F.), n=11; Group B: STZ+SS-20 10 mg/kg s.c. q.d. (M.-F.), n=11; Group C: STZ+ vehicle s.c. q.d. (M.-F.), n=10; Group D: NRC + vehicle s.c. q.d. (M.-F.), n=10. The therapeutic schedule is shown in Table 13.

**TABLE 13. Therapeutic schedule**

| **Duration** | **Objective** | **Diabetic groups (A,B,C)** | **Control group (D)** |
|---|---|---|---|
| 1^{st}-3^{rd} week | Acclimation | Normal rat chow | |
| End of 3^{rd} week | STZ injection | STZ 65 mg/kg, i.p., once | Citrate buffer 2 mL/kg, i.p., once |
| 4^{th} - 18^{th} week | Induction of diabetes | normal rat chow | |
| 19^{th} - 28^{th} week | Peptide treatment | GroupA:MTP-131 10mg/kg/day | Group D: |
| | | Group B:SS-20 10 mg/kg/day | Saline 2 mL/kg/day |
| | | Group C: Saline 2 mL/kg/day | s.c., 5 days per week |
| | | s.c., 5 days per week | |
| 29^{th}week | Harvested organs and tissues | | |

The compounds were dissolved in saline. The treatment was given once a day (5 days a week) with injection site to be alternated. The concentration of SS-31 and SS-20 dosed at 10mg/kg is 5mg/ml. The treatment volume (0.2 ml/100 g for s.c.) for each test animal was adjusted according to its body weight taken on the beginning of each week. STZ was dissolved in 0.1 M citrate buffer (pH4.5) to concentration of 20 mg/mL. STZ was injected intraperitoneally.

FIG. 6 shows that TG level in SS-31 and SS-20 groups increased less than STZ/Saline group after peptides treatment for 2 weeks or 4 weeks. It indicates that SS-31 and SS-20 have effects in regulating lipid metabolism.

### Example 5 - Prevention and Treatment of Metabolic Syndrome by Aromatic Cationic Peptides in the Zucker Rat Model

To further demonstrate the prevention of metabolic syndrome on the one hand, and treatment of metabolic syndrome on the other hand, the aromatic-cationic peptides of the invention are tested on the fatty (fa/fa) Zucker rat, a model of diet-induced metabolic syndrome. Compared with the 6 wk high fat fed Sprague-Dawley rat model (as used in Example 1), the fatty Zucker rats develop a much greater degree of obesity and insulin resistance.

First, to examine the effects of the aromatic-cationic peptides on prevention of metabolic syndrome, young Zucker rats (~3-4 weeks of age) are administered an aromatic-cationic peptide (e.g., SS-20 or SS-31) for approximately 6 weeks. As these young rats do not yet exhibit signs or symptoms of metabolic syndrome, they provide a useful model for assessing the efficacy of methods of preventing metabolic syndrome. SS-31 or SS-20 (1.0-5.0 mg/kg body wt) is administered to the rats *via* i*.*p*.* or oral administration (*i.e*., drinking water or gavage).

It is predicted that SS-31 administration will attenuate or prevent the symptoms of metabolic syndrome that normally develops in the fatty (fa/fa) Zucker rat. Measured outcomes include body weight, fasting glucose/insulin/free fatty acid, glucose tolerance (OGTT), *in vitro* muscle insulin sensitivity (incubation), markers of insulin signaling (Akt-P, IRS-P), mitochondrial function studies on permeabilized fibers (respiration, H₂O₂ emission), markers of intracellular oxidative stress (lipid peroxidation, GSH/GSSG ratio, aconitase activity) and mitochondrial enzyme activity. A comparison is made between control rats and fa/fa rats administered SS-31. Controls include wild-type and fa/fa rats not administered SS-31. Successful prevention of metabolic syndrome by the aromatic-cationic peptides of the invention is indicated by a reduction in one or more of the markers associated with metabolic syndrome enumerated above.

Second, to examine the effects of the aromatic-cationic peptides of the invention on treatment of metabolic syndrome, Zucker rats (∼12 weeks of age) are administered SS-31 for approximately 6 weeks. As these rats show signs of obesity and metabolic syndrome, they provide a useful model for assessing the efficacy of methods of treating insulin resistance. SS-31 (1.0-5.0 mg/kg body wt) is administered to the rats *via* i.p. or oral administration (*i.e*., drinking water or gavage).

It is predicted that SS-31 administration will ameliorate or reduce metabolic syndrome that normally develops in these older fatty (fa/fa) Zucker rats. Measured outcomes include body weight, fasting glucose/insulin/free fatty acid, glucose tolerance (OGTT), *in vitro* muscle insulin sensitivity (incubation), markers of insulin signaling (Akt-P, IRS-P), mitochondrial function studies on permeabilized fibers (respiration, H₂O₂ emission), markers of intracellular oxidative stress (lipid peroxidation, GSH/GSSG ratio, aconitase activity) and mitochondrial enzyme activity. A comparison is made between control rats and fa/fa rats administered SS-31. Controls include wild-type and fa/fa rats not administered SS-31. Successful treatment of metabolic syndrome by the aromatic-cationic peptides of the invention is indicated by a reduction in one or more of the markers associated with metabolic syndrome enumerated above.

### EQUIVALENTS

The present invention is not to be limited in terms of the particular embodiments described in this application, which are intended as single illustrations of individual aspects of the invention. Many modifications and variations of this invention can be made without departing from its spirit and scope, as will be apparent to those skilled in the art. Functionally equivalent methods and apparatuses within the scope of the invention, in addition to those enumerated herein, will be apparent to those skilled in the art from the foregoing descriptions. Such modifications and variations are intended to fall within the scope of the appended claims. The present invention is to be limited only by the terms of the appended claims, along with the full scope of equivalents to which such claims are entitled. It is to be understood that this invention is not limited to particular methods, reagents, compounds compositions or biological systems, which can, of course, vary. It is also to be understood that the terminology used herein is for the purpose of describing particular embodiments only, and is not intended to be limiting.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc.* As a non-limiting example, each range discussed herein can be readily broken down into a lower third, middle third and upper third, *etc.* As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

All patents, patent applications, provisional applications, and publications referred to or cited herein are incorporated by reference in their entirety, including all figures and tables, to the extent they are not inconsistent with the explicit teachings of this specification.

Other embodiments are set forth within the following claims.

### Clauses:

Aspects and embodiments of the present invention may also be provided according to the following numbered paragraphs.
1. A method of treating or preventing metabolic syndrome in a mammalian subject, comprising administering to the mammalian subject in need thereof a therapeutically effective amount of the peptide D-Arg-2'6'-Dmt-Lys-Phe-NH₂ or Phe-D-Arg-Phe-Lys-NH₂.
2. The method of paragraph 1, wherein the metabolic syndrome is associated with resistance to insulin-mediated glucose uptake, glucose intolerance, hyperinsulemia, increased LDL-cholesterol, increased VLDL, increased triglycerides, decreased HDL-cholesterol, increased plasminogen activator inhibitor-1 (PAI-1) levels or hypertension.
3. The method of paragraph 1, wherein the metabolic syndrome is characterized by three or more of the following criteria:
   (a) abdominal obesity: waist circumference >102 cm in men and >88 cm in women;
   (b) hypertriglyceridemia: ≥ 150 mg/dl (1.695 mmol/l);
   (c) low HDL cholesterol: <40 mg/dl (1.036 mmol/l) in men and <50 mg/dl (1.295 mmol/l) in women;
   (d) high blood pressure: ≥ 130/85 mm Hg; and
   (e) high fasting glucose: ≥ 1 10 mg/dl (>6.1 mmol/l).
4. The method of paragraph 1, wherein the metabolic syndrome is characterized by diabetes, impaired glucose tolerance, impaired fasting glucose, or insulin resistance plus two or more of the following abnormalities:
   (a) high blood pressure: ≥ 160/90 mm Hg;
   (b) hyperlipidemia: triglyceride concentration > 150 mg/dl (1.695 mmol/l) and/or HDL cholesterol <35 mg/dl (0.9 mmol/l) in men and <39 mg/dl (1.0 mmol/l) in women;
   (c) central obesity: waist-to-hip ratio of >0.90 in men or >0.85 in women or BMI >30 kg/m²; and
   (d) microalbuminuria: urinary albumin excretion rate >20 µg/min or an albumin to creatinine ratio ≥ 20 mg/g.
5. The method of paragraph 1, wherein the metabolic syndrome is characterized by three or more of the following criteria:
   (a) triglycerides >150 mg/dl;
   (b) systolic blood pressure (BP) ≥ 130 mm Hg or diastolic blood pressure ≥ 85 mm Hg or on antihypertensive treatment;
   (c) high-density lipoprotein cholesterol <40 mg/dl;
   (d) fasting blood sugar (FBS) >1 10 mg/dl; and
   (e) body mass index > 28.8 kg/m².
6. The method of paragraph 1, wherein the metabolic syndrome is treated through improvement of lipid metabolism compared to the subject's lipid metabolism before being administered the peptide.
7. The method of paragraph 6, wherein the improvement in lipid metabolism is reduction of blood triglyceride level compared to the subject's blood triglyceride level before being administered the peptide.
8. The method of paragraph 6, wherein the improvement in lipid metabolism is improvement of blood HDL/LDL cholesterol ratio compared to the subject's blood HDL/LDL cholesterol before being administered the peptide.
9. The method of paragraph 1, wherein the metabolic syndrome is treated by reducing blood sugar level compared to the subject's blood sugar level before being administered the peptide.
10. The method of paragraph 1, wherein the metabolic syndrome is treated by a reduction in body weight compared to the subject's body weight before being administered the peptide.
11. The method of paragraph 1, wherein the subject is a human.
12. The method of paragraph 1, wherein the peptide is administered prior to the onset of type II diabetes.
13. The method of paragraph 1, wherein the peptide is administered orally, systemically, intravenously, subcutaneously, or intramuscularly.
14. The method of paragraph 1 further comprising the step of identifying the mammal in need of treating or preventing metabolic syndrome.
15. The method of paragraph 1 further comprising the step of monitoring the mammal for treatment or prevention of metabolic syndrome.

## Claims

1. A peptide Phe-D-Arg-Phe-Lys-NH₂ for use in treating obesity in a subject in need thereof.

2. The peptide for use according to claim 1, wherein the obesity is diet-induced.

3. The peptide for use according to claim 1, wherein the obesity is abdominal obesity **characterized by** a waist circumference of > 102 cm in men and > 88 cm in women.

4. The peptide for use according to claim 1, wherein the obesity is central obesity **characterized by** a waist-to-hip ratio of > 0.90 in men or > 0.85 in women or a BMI of > 30 kg/m².

5. The peptide for use according to claim 1, wherein the obesity is treated by reducing the subject's abdominal fat index.

6. The peptide for use according to claim 1, wherein the obesity is treated by reducing the subject's subcutaneous fat index.
